(19) Europäisches Patentamt
European Patent Office
Office européen des brevets

(11) EP 1 717 247 A1

(12) EUROPEAN PATENT APPLICATION

(43) Date of publication:
02.11.2006 Bulletin 2006/44

(21) Application number: 05009361.6

(22) Date of filing: 28.04.2005

(51) Int Cl.:
*C07K 14/655* (2006.01) *C07K 7/64* (2006.01)
*A61K 38/12* (2006.01) *A61K 51/00* (2006.01)

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(71) Applicant: **Schering AG**
**13342 Berlin (DE)**

(72) Inventors:
- **Srinivasan, Ananth**
  **10629 Berlin (DE)**
- **Luyt, Leonard G.**
  **London, ON N6C1L6 (CA)**

(74) Representative: **Krauss, Jan et al**
**Forrester & Boehmert,**
**Pettenkoferstrasse 20-22**
**80336 München (DE)**

# (54) Cyclic peptides binding to the somatostatin receptor

(57) The present invention concerns compounds comprising novel cyclized peptides with increased selectivity towards binding to the somatostatin receptor 5 ($SSTR_5$) its production and use in the diagnosis and treatment of somatostatin-responsive diseases or diseases characterized by up-regulation of somatostatin receptors, in particular proliferative diseases.

EP 1 717 247 A1

## Description

The present invention concerns compounds comprising novel cyclized peptides with increased selectivity towards binding to the somatostatin receptor 5 ($SSTR_5$) its production and use in the diagnosis and treatment of somatostatin-responsive diseases or diseases characterized by up-regulation of somatostatin receptors, in particular proliferative diseases.

Cancer is the leading cause of morbidity and mortality in developed countries. For example, approximately 1.4 million new cases and more than 0.5 million cancer deaths were reported in the U.S. in 1996. In 1995, the total annual cost of cancer care in the U.S., including direct and indirect costs, was estimated to be more than $ 96 billion. A great need exists for improved diagnostic and therapeutic tools to allow early detection and safe, cost-effective treatment of cancer.

Many tumors express receptors for the peptide hormone somatostatin. In particular, neuroen-docrine tumors such as pituitary adenomas, pheochromocytomas, paragangliomas, some medullary thyroid carcinomas, and some small cell lung cancers express somatostatin receptors (SSTRs). In addition cells of nervous system tumors such as astrocytomas und meningeomas display SSTRs on their surfaces. SSTR expression has also been found in human breast tumors, malignant lymphomas and renal cell carcinomas. In addition some prostate tumors may be characterized by SSTR expression.

Binding studies performed with radiolabelled or iodinated somatostatin and its analogues have identified five SSTR subtypes ($SSTR_{1-5}$). The SSTR bearing tumors described above express $SSTR_2$ and $SSTR_5$ most frequently, with $SSTR_3$ and $SSTR_4$ occurring less frequently, according to most authors. One group reports that $SSTR_3$ is expressed at very high levels in almost all human tumors (Virgolini (1997) Eur. J. Clin. Invest. 27, 793-800). There is a general agreement that most tumors typically express more than one SSTR subtype, and that varying intensities of SSTRs may be expressed in cells contained within a particular tumor.

Until recently, three somatostatin analogues have been commercially available. Octreotide (Sandostatin®) binds to $SSTR_2$, $SSTR_3$ and $SSTR_5$ and is marketed in the U.S. and Europe for treatment of acromegaly and control of symptoms associated with vipomas and metastatic 49.393 carcinoid tumors. Lanreotide (Somatuline™) has a SSTR subtype profile similar to that of Octreotide and is approved in several European countries for the same indications as Octreotide. A radiolabelled form of Octreotide, $^{111}$In-pentetreotide ($^{111}$In-DTPA-D-Phe$^1$-Octreotide or $^{111}$In-OctreoScan®) has been approved in the U.S. and in Europe for imaging neuroendo-crine tumors. Recently, the U.S. FDA approved a new radio pharmaceutical, NeoTect™, a $^{99m}$Tc-labelled form of the novel somatostatin analogue depreotide (P829), for sale as an imaging agent. Bloom, et al. (1999), Chest. 115: 224-232, describes the use of $^{99m}$Tc-depreotide for evaluation of solitary pulmonary nodules of the lung. $^{99m}$Tc-labelled depreotide has also been studied as an imaging agent for other somatostatin-receptor bearing tumors. Depreotide is described in WO 95/00553 and WO 95/33497.

The art known somatostatin analogues do, thus, not exhibit a significant selectivity for either $SSTR_2$ or $SSTR_5$ and only a limited number of ligands have been found to impart preferential binding to one of the somatostatin subtypes. For non-peptido structures, agonists for each of the five subtypes were reported (Rohrer, S.P. et al. (1998) Science 282: 737-740). A backbone-cyclic somatostatin analogue has also been reported to have some $SSTR_5$ selectivity (Gilon, C. (1998) J. Med. Chem. 41: 919-929). Other backbone-cyclic hexapeptides that bind with nanomolar and even subnanomolar affinity to the somatostatin receptor (US 6,183,722 and WO 01/044177) have been described. These entities typically show a preference for $SSTR_2$ over the other subtypes.

Because of the differential distribution of the different subtypes in various tissues and diseased tissues it is desirable to develop binding compounds which show an increased subtype selectivity, while maintaining a high affinity in the nanomolar range. The present inventors have now surprisingly identified new constrained backbone-cyclized peptides which improved binding to somatostatin receptor subtype 5 and which simultaneously show a decreased somatostatin subtype 2 binding activity, i.e. which have an increased specificity to $SSTR_5$, if compared to the somatostatin receptor agonist ReP2045. The novel compounds are therefore somatostatin analgoues with an improved selectivity towards somatostatin receptor subtype 5.

### Detailed Description of the Invention

A first aspect of the present invention concerns a compound comprising a cyclized peptide having the formula (I)

$$\text{cyclo}[X^3\text{-DTrp-Lys-}X^4\text{-}X^5\text{-}X^6] \qquad (I),$$

wherein

$X^3$ is selected from the group consisting of diphenyl-Ala, (1)Nal, (2)Nal, (4)Pal, Phe(4-F), Thioproline, Trp and Tyr;
$X^4$ is selected from the group consisting of βAla(cyclopropyl), diaminopropanoic acid (Dpr), Thr and Val;

$X^5$ is an amino acid containing a side-chain, capable of forming a direct or indirect bond to a metal chelating residue, a polypeptide, a drug or a dye; a natural amino acid; or an unnatural amino acid,

$X^6$ an amino acid containing a side-chain, capable of forming a direct or indirect bond to a metal chelating residue, a therapeutic or a dye; a natural amino acid; or an unnatural amino acid

under the proviso that $X^5$ is cysteine, homo-cystein or methionine, when $X^3$ has the meaning Tyr and $X^4$ has the meaning Thr. This cyclized peptides show an increased binding to SSTR5, if compared to the prior art somatostatin receptor agonist binding compound ReP2045 which has a sequence cyclo [Tyr-DTrp-Lys-Thr-Phe-(NMe)hCys]-$CH_2$CO-β-D-Pr-Phe(4-$NH_2$-Cys-Thr-Ser-OH-[R=O]). The affinities of the cyclized peptides of the present invention to the respective SSTR5 and SSTR2 can be determined by art known methods described for example, in Gazal S. et al. (2002) J. Med. Chem 45: 1665-1671. On one hand the absolute affinity to SSTR5 is important for increasing the targeting of SSTR5 and on the other hand the relative ratios of affinity towards SSTR2 versus affinity towards SSTR5. Preferably the cyclized peptides of the present invention show an $IC_{50}$ in nM towards SSTR5 of less than 50, preferably less than 45, more preferably of less than 40, less than 35, less than 30, less than 25, less than 20, less than 15, less than 10. At the same time it is preferred that the $IC_{50}$ in nM towards SSTR2 is larger than 0.1, larger than 0.2, larger than 0.4, larger than 0.5, larger than 0.6, larger than 0.7, larger than 0.8, larger than 0.9, larger than 1.0, larger than 1.5, larger than 2.0, larger than 2.5, larger than 3.0, larger than 3.5, larger than 4.0 and larger than 5.0. The ratio of SSTR2 to SSTR5 affinity in % is 0.014 (ratio 2/5 in %) for ReP2045, i.e. the binding affinity of ReP2045 to SSTR5 is only 0.014% of its binding affinity to SSTR2. The cyclized peptides of the present invention show at least a 5-fold improvement of that ratio. In a preferred embodiment the ratio of the IC50 of SSTR2/SSTR5 in % is at least 0.2, 0,3, 0.4, 0.5, 0.6, 0.7, 0.8, 0.9, 1.0, 1.1, .1.2, 1.3, 1.4, 1.5, 1.6, 1.7, 1.8, 1.9, 2.0, 2.5, 3.0, 3.5, 4.0, 4.5, 5.0, 5.5, 6.0, 6.5, 7.0, 7.5, 8.0, 8.5, 9.0, 9.5, 10.0, 11, 12, 13, 14, 15, 16, 17, 18, 19, 20, 25, 30, 35, 40, 45, 50, 55, 60, 65, 70, 75, 80, 85, 90, 95, or 100, i.e. it is particular preferred that the cyclized peptides have a binding affinity to SSTR5, which is equal to SSTR2. In the most preferred embodiments the binding affinity to SSTR5 is higher than to SSTR2, e.g. preferably 150%, 200%, 250%, 300%, or 350%.

In one embodiment at least one of the amino acids of $X^3$, $X^4$, $X^5$, or $X^6$, preferentially at least one of the amino acid of $X^5$ or $X^6$ comprise at least one halogen moiety, preferably attached to a side chain.

In a preferred embodiment of the compound of the present invention $X^3$ is selected from the group consisting of Tyr and (1)Nal. It is further preferred that $X^4$ is selected from the group consisting of Thr and Val and it is particular preferred that $X^3$ has the meaning Tyr or (1)Nal and $X^4$ has the meaning Thr and Val.

Preferably, the compounds of the present invention only comprise one direct or indirect bond to a metal chelating residue, a polypeptide, a drug or a dye and, consequently, it is preferred that either $X^5$ or $X^6$ is an amino acid containing a side-chain capable of forming a direct or indirect bond to metal chelating residue, a polypeptide, a drug or a dye. An "amino acid containing a side-chain, capable of forming a direct or indirect bond" is an amino acid residue with a side-chain that carries a functional group which can be activated to form a bond to another residue or which can be attacked by an activated residue on another molecule to form a covalent bond. Preferably such reactive groups are thio, hydroxy, carboxy or amino residues. It is particular preferred that the side-chain comprises at least one thio group and, therefore, particular preferred amino acids cysteine, homo-cysteine and methionine and in particular methionine. A further preferred amino acid is Lys(GlyMeDOTA).

The term "direct bond" in this context and as used throughout the specification means a covalent bond to a further residue, i.e. a direct bond to a metal chelating residue, a polypeptide, a drug or a dye, while the term "indirect bond" as used herein means that one or more additional chemical residues, which are attached via covalent or non-covalent bonds are located between the amino acid $X^5$ and/or $X^6$ and the metal chelating residue, the polypeptide, the drug or the dye. These one or more additional chemical residues can also be termed "spacer". A spacer can, e.g. provide a spatial separation between the constrained backbone cyclized peptides of the present invention and the further functionality of the compound which is coupled to the compound through the amino acid containing a side-chain, capable of forming a direct or indirect bond.

In a preferred embodiment $X^5$ is an amino acid containing a side-chain capable of forming a direct or indirect bond to a metal chelating residue, a polypeptide, a drug or a dye and $X^6$ is a natural amino acid or a unnatural amino acid.

Within the context of the present invention $X^5$ and/or $X^6$ can mean any naturally occurring amino acids. These amino acids may be referenced herein in abbreviated form, using standard one-letter or three-letter codes (which can be found, for example, in G. Zubay, Biochemistry (2d. ed.), 1988 (MacMillen Publishing: New York) p.33). In addition, as used herein, the following amino acids and amino acid analogues are intended to be represented by the following abbreviations: Hcy is homocysteine; Hhc is homohomocysteine (3-mercaptopropylglycine); Pen is penicillamine; Aib is aminoisobutyric acid; Nal is 2-naphthylalanine; Aca is 6-aminocaproic acid; Ain is 2-aminoindan-2-carboxylic acid; Hly is homolysine; Achxa is 4-amino-cyclohexylalanine; Amf is 4-aminomethyl-phenylalanine; Aec is S-(2-aminoethyl) cysteine; Apc is S-(3-aminopropyl) cysteine; Aes is O-(2-aminoethyl)serine; Aps is O-(3-aminopropyl)serine; Abu is 2-aminobutyric acid; Nva is norvaline; $F_D$ is D-phenylalanine; $W_D$ is D-tryptophan; $Y_D$ is D-tyrosine; Cpa is L-(4-chlorophenyl) alanine; Thp is 4-amino-tetrahydrothiopyran-4-carboxylic acid; D-Nal is D-2-naphthylalanine; Dpg is dipropylglycine; Nle

is norleucine; (N-$CH_3$)Cys is N-methyl-cysteine; (N-$CH_3$)Hcy is N-methyl-homocysteine; (N-$CH_3$)Tyr is N-methyl-tyrosine; (N-$CH_3$)Tty is N-methyl-thiotyrosine (i.e., N-methyl-4-mercaptophenylalanine); (N-$CH_3$)Tyr($CH_2$ $CH_2$ SH) is N-methyl-O-2-mercaptoethyl tyrosine; Thr(OH) is threoninol residue (wherein the carboxyl group of the amino acid is reduced to a primary alcohol, incorporated into the peptide using the procedure of Neugebauer et al. (1990, Peptides: Proceedings of the 11th American Peptide Symposium, pp. 1020-21); Ser(ol) is. serinol; Asp(ol) is aspartinol; Glu(ol) is glutarinol; Gln(ol) is glutaminol; Asn(ol) is asparaginol; Phe(4-F) is 4-fluoro-phenylalanine; Phe(4-$NH_2$) is 4-amino-phenylalanine; ε-Lys represents a covalent linkage via the ε-amino group on the side chain of a lysine residue; δ-Orn represents an ornithine residue in which the δ-amino group is covalently linked to the carboxyl group of the adjacent amino acid to form a peptide bond; γ-Dab represents a 2,4-diaminobutyric acid residue in which the γ-amino group is covalently linked to the carboxyl group of the adjacent amino acid to form a peptide bond; β-Dap represents a 2,3-diaminopropionic acid residue in which the β-amino group is covalently linked to the carboxyl group of the adjacent amino acid to form a peptide bond. When a combination of one-letter codes is used with the abbreviations set forth above, the abbreviation is set off by periods. The term "non naturally occurring amino acids" also comprise substituted derivatives of natural occurring amino acids

In accordance with the present invention, a "substituted derivative" of an amino acid includes such substitutions as amino, hydroxyl, N-alkyl wherein alkyl represents $C_1$ to $C_4$ alkyl, N-aryl, N-acyl, O-alkyl wherein alkyl represents $C_1$ to $C_4$ alkyl, O-aryl, O-acyl, S-alkyl wherein alkyl represents $C_1$ to $C_4$ alkyl, S-aryl. When applied to amino acids that contain a side chain aromatic ring, the term also encompasses o-, m-, and p-substitutions including but not limited to o-amino, m-amino, p-amino, amino, o-hydroxyl, m-hydroxyl, p-hydroxyl, and the like.

Particularly preferred natural or unnatural amino acids which can constitute $X^5$ or $X^6$, and which preferably constitute $X^6$ are alanine, asparagine, aspartic acid, glutamine, glutamic acid, phenylalanine, glycine, histidine, isoleucine, lysine, leucine, proline, arginine, serine, threonine, tryptophane, valine, tyrosine, tert-butyl glycine, N-methyl phenylalanine (NMe)Phe, Hcy, Hhc, Pen, Aib, Nal, Aca, Ain, Hly, Achxa, Amf, Aec, Apc, Aes, Aps, Abu, Nva, FD, WD, YD, Cpa, Thp, D-Nal, Dpg, Nle, (1)Nal, (2)Nal, (N-$CH_3$)Cys, (N-$CH_3$)Hcy, (N-$CH_3$)Tyr, (N-$CH_3$)Tty, (N-$CH_3$)Tyr($CH_2CH_2$SH), Tpi, Thr(OH), Ser(ol), Asp(ol), Glu(ol), Gln(ol), Asn(ol), (4)PaL, Phe(4-F), Phe(4-$NH_2$), ε-Leusine, δ-Orn, γ-Dab and β-Dab. Out of those amino acids (NMe)Phe, Phe and Tpi are particularly preferred amino acids. An increased binding to $SSTR_5$ has been observed when $X^6$ is selected from the group consisting of (NMe)Phe, Phe and Tpi.

Furthermore, within the context of the preferred meaning of $X^6$ it is preferred that $X^5$ is selected from the group consisting of cysteine, homo-cysteine, methionine and Lys(GlyMeDOTA).

Particularly preferred compounds of the present invention, which show an increased affinity towards $SSTR_5$ while at the same time having a reduced binding to $SSTR_2$ (if compared to the somatostatin receptor agonist binding compound ReP2045 which has a sequence cyclo [Tyr-DTrp-Lys-Thr-Phe-(NMe)hCys]-$CH_2$CO-O-D-Pr-Phe(4-$NH_2$-Cys-Thr-Ser-OH-[R=O]) have been identified. In those compounds $X^3$, $X^4$, $X^5$ and $X^6$ have the meaning as indicated below

a) cyclo[(1)Na1-DTrp-Lys-Thr-Met-(NMe)Phe];
b) cyclo[Trp-DTrp-Lys-Thr-Met-(NMe)Phe];
c) cyclo[(1)Nal-DTrp-Lys-Val-Met-(NMe)Phe];
d) cyclo[Phe(4-F)-DTrp-Lys-Thr-Met-(NMe)Phe];
e) cyclo[Tyr-DTrp-Lys-Val-Met-(NMe)Phe];
f) cyclo[(1)Na1-DTrp-Lys-Thr-Lys(GIyMeDOTA)-(NMe)Phe];
g) cyclo[Tyr-DTrp-Lys-Thr-Met-(NMe)Phe];
h) cyclo[(2)Nal-DTrp-Lys-Thr-Met-(NMe)Phe];
i) cyclo[Tyr-DTrp-Lys-Thr-Met-Tpi];
j) cyclo[Tyr-DTrp-Lys-BAIa(cyclopropyl)-Met-(NMe)Phe];
k) cyclo[Tyr-DTrp-Lys-Dpr-Met-(NMe)Phe];
l) cyclo[ThioPro-DTrp-Lys-Thr-Met-Phe];
m) cyclo[DiphenylAla-DTrp-Lys-Thr-Met-(NMe)Phe];
n) cyclo[(4)Pal-DTrp-Lys-Thr-Met-(NMe)Phe].

As had been set out above somatostatin receptors are more abundant in certain diseased tissues, in particular in proliferating tissue and, therefore, the constrained backbone cyclized peptides of the present invention, which bind to somatostatin receptors can be used to recruit a variety of different compounds to cells and tissues which express somatostatin receptors and in particular to cells and tissues, which show an increased expression of somatostatin receptor 5 subtype over somatostatin receptor 2 subtype. Such compounds include metal chelating residues comprising a metal, polypeptides, drugs and dyes. In addition or alternatively the preferred somatostatin binding compounds are modified to comprise at least one halogen. In this respect it is preferred that the amino acids at position $X^3$, $X^4$, $X^5$, or $X^6$, preferentially at least one of the amino acid at position $X^5$ or $X^6$ comprise a halogen, preferably attached to a side chain.

The metal chelating residue serve the purpose of binding to metals in particular to metal ions and to recruit such metal ions to the tissues showing expression of $SSTR_5$, in particular an increased expression of $SSTR_5$. A large variety of such metal chelating moieties are known in the art and are described in, for example, US 5,654,272, US

5,681,541, US 5,788,960, US 5,811,394, US 5,720,934, US 5,776,428, US 5,780,007, US 5,922,303, US 6,093,383, US 6,086,849, US 5,965,107, US 5,300,278, US 5,350, 837, US 5,589,576, US 5,679,778, US 5,789,659 and US 6,358,491. In a preferred compound of the present invention the metal chelating residue, which is connected through a direct or indirect bond to either the amino acid side-chain of $X^5$ or $X^6$, preferentially to the amino acid side chain of $X^5$, is selected from the group consisting of:

a) $C(pgp)^s$-(aa)-$C(pgp)^s$, wherein $(pgp)^s$ is hydrogen or a thiol protecting group and (aa) is any [alpha]- or [beta]-amino acid not comprising a thiol group;

b) a substance according to formula (II) or (III)

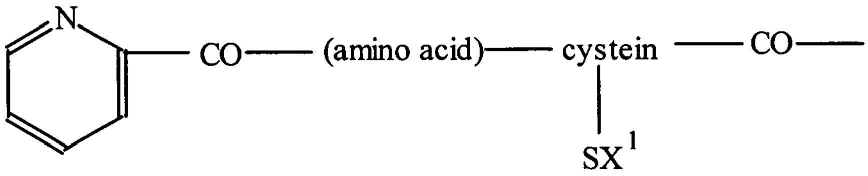

(II)

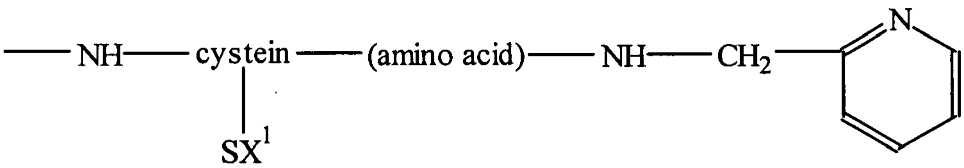

(III),

wherein X'=H or a protecting group;
(amino acid)=any amino acid;

c) a substance according to formula (IV)

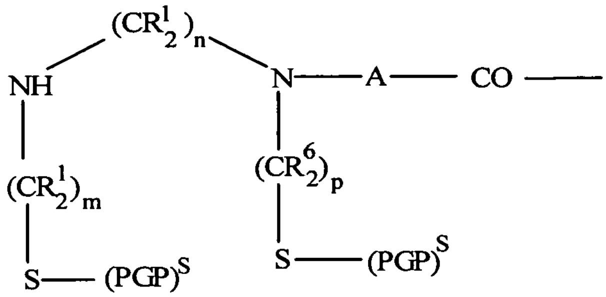

wherein each $R^1$ is independently H, $CH_3$ or $C_2H_5$, each $(PGP)^s$ is independently a thiol protecting group or H; m, n and p are independently 2 or 3; A is linear $C_1$-$C_8$ alkyl, e.g. methyl, ethyl, propyl, isopropyl, n-butyl, isobutyl, pentyl, hexyl, heptyl or octyl, substituted linear $C_1$-$C_8$ alkyl, e.g. substituted methyl, ethyl, propyl, isopropyl, n-butyl, isobutyl, pentyl, hexyl, heptyl or octyl, cyclic $C_3$-$C_8$ alkyl, e.g. cyclic propyl, butyl, pentyl, hexyl, heptyl or octyl, substituted cyclic $C_3$-$C_8$ alkyl, e.g. substituted cyclic propyl, butyl, pentyl, hexyl, heptyl or octyl, aryl, substituted aryl, or a combination thereof; and

d) a substance according to formula (V)

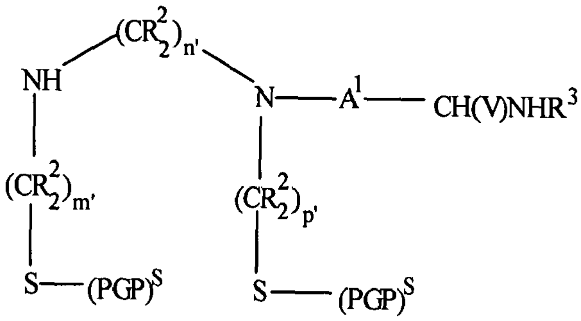

(V),

wherein each $R^2$ is independently H, $CH_3$ or $C_2H_5$; each $(PGP)^s$ is independently a thiol protecting group or H; m', n' and p' are independently 2 or 3; $A^1$ is linear $C_1$-$C_8$ alkyl, e.g. methyl, ethyl, propyl, isopropyl, n-butyl, isobutyl, pentyl, hexyl, heptyl or octyl, substituted linear $C_1$-$C_8$ alkyl, e.g. substituted methyl, ethyl, propyl, isopropyl, n-butyl, isobutyl, pentyl, hexyl, heptyl or octyl, cyclic $C_3$-$C_8$ alkyl, e.g. cyclic propyl, butyl, pentyl, hexyl, heptyl or octyl, substituted cyclic $C_3$-$C_8$ alkyl, e.g. substituted cyclic propyl, butyl, pentyl, hexyl, heptyl or octyl, aryl, substituted aryl, or a combination thereof; V is H or a CO link to $X^5$ or $X^6$; $R^3$ is H or covalent link to $X^5$ or $X^6$;

e) diethylenetriaminepentaacetic acid (DTPA);

f) a derivative of DTPA having a formula (VI)

$$(HOOCCH_2)_2N(CR^4_2)(CR^4_2)N(CH_2COOH)(CR^4_2)(CR^4_2)N(CH_2COOH)_2 \quad (VI),$$

wherein each $R^4$ is independently H, $C_1$ to $C_4$ alkyl, or aryl and one $R^4$ is linked to $X^5$ or $X^6$;

g) ethylenediaminetetraacetic acid (EDTA);

h) a derivative of EDTA having a formula (VII)

$$(HOOCCH_2)_2N(CR^5_2)(CR^5_2)N(CH_2COOH)_2 \quad (VII),$$

wherein each $R^5$ is independently H, $C_1$ to $C_4$ alkyl, or aryl and one $R^5$ is covalently linked to $X^5$ or $X^6$;

i) 1,4,7,10-tetraazacyclododecanetetraacetic acid and derivatives thereof;

j) a substance according to formula (VIII)

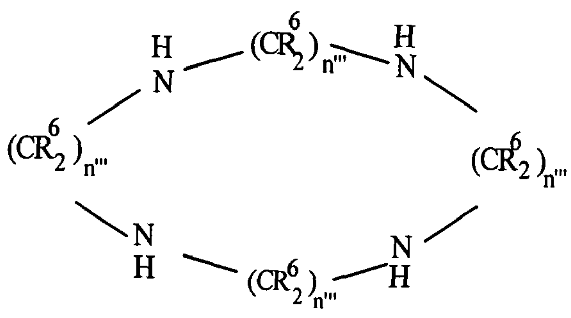

(VIII),

wherein n"' is an integer that is 2 or 3 and where each $R^6$ is independently H, $C_1$ to $C_4$ alkyl, or aryl and one $R^6$ is covalently linked to $X^5$ or $X^6$;

k) a substance according to formula (IX) comprising a single thiol

$$A^2\text{-}CZ^2\text{-}(B^2)\text{-}\{C(R^7R^8)\}_{n''}\text{-}X^2 \qquad (IX),$$

wherein $A^2$ is H, HOOC-, $H_2NOC$-, -NHOC-, -OOC-, $R_2^{11}NOC$-, $X^2$-NHOC-, $X^2$-OOC-, or $R^9$; $B^2$ is H, SH, -$NHR^{10}$, -$N(R^{10})$-, $X^2$-$NR^{10}$- or $R^9$; $Z^2$ is H or $R^{10}$; $X^2$ is SH, -$NHR^{10}$, -$N(R^{10})$-, $X^2$-$NR^{10}$- or $R^7$; $R^8$, $R^9$ and $R^{10}$ are independently H, straight chain $C_1$-$C_8$ alkyl, e.g. methyl, ethyl, propyl, isopropyl, n-butyl, isobutyl, pentyl, hexyl, heptyl or octyl, branched chain $C_1$-$C_8$ alkyl, e.g. methyl, ethyl, propyl, isopropyl, n-butyl, isobutyl, pentyl, hexyl, heptyl or octyl, or cyclic $C_3$-$C_8$ alkyl, e.g. cyclic propyl, butyl, pentyl, hexyl, heptyl or octyl; n" is 0, 1 or 2; $R^{11}$ is $C_1$-$C_4$ alkyl, an amino acid, or a peptide comprising 2 to about 10 amino acids; and: (1) where $B^2$ is -$NHR^{10}$, X-$NR^{10}$- or -$N(R^{10})$-, $X^2$ is SH and n" is 1 or 2; (2) where $X^2$ is -$NHR^{10}$, $X^2$-$NR^{10}$-, or -$N(R^{10})$-, $B^2$ is SH and n" is 1 or 2; (3) where $B^2$ is H or R9, $A^2$ is HOOC-, $H_2NOC$-, X-NHOC-, X-OOC-, -NHOC-, or -OOC-, $X^2$ is SH and n" is 0 or 1; (4) where $A^2$ is H or $R^9$, then where $B^2$ is SH, $X^2$ is - $NHR^{10}$, $X^2$-$NR^{10}$-, or -$N(R^{10})$- and where $X^2$ is SH, $B^2$ is -$NHR^{14}$, $X^2$-$NR^{10}$- or - N $(R^{10})$ and n" is 1 or 2; (5) where $X^2$ is H or $R^{10}$, $A^2$ is HOOC-, $H_2NOC$-, - NHOC-, -OOC-, $X^2$-NHOC- or $X^2$-OOC- and $B^2$ is SH; and (6) where $Z^2$ is methyl, $X^2$ is methyl, $A^2$ is HOOC-, $H_2NOC$-, -NHOC-, -OOC-, $X^2$-NHOC- or $X^2$-OOC- and $B^2$ is SH and n" is 0.

A particularly preferred chelator, which can be directly or indirectly bound to a cyclized peptide of the present invention is a substance according to formula (X)

$$\beta\text{-Dap-Xaa-Cys-Zaa-A} \qquad (X),$$

wherein Xaa is an L-α-amino acid;
Zaa is an α-amino acid, an α-amino acid amide, an aminoethylether, a β-aminol, or a peptide containing from two to ten α-amino acids, said peptide having a carboxyl terminal α-amino acid, α-amino acid amide, aminoethylether, or β-aminol, and A is the amino or carboxyl group of the amino acid, or a protected amino or carboxyl group. Preferably this metal chelator is bound via the amino or carboxyl group to the cyclized peptides of the present invention. Homologs of β-Dap may also be employed in the compounds of the present invention.

Suitable L-α-amino acids for substitution as Xaa in the preferred chelators of the invention include naturally occurring amino acids such as asparagine, glutamine, threonine, serine, arginine, histidine, lysine, ornithine, phenylalanine, tyrosine, and, in addition, synthetic amino acids containing hydrophilic substituents. Exemplary synthetic amino acids include, without limitation, diaminopropionic acid; diaminobutyric acid; substituted tyrosines such as haloty-rosine; hydroxyltyrosine; aminotyrosine; substituted phenylalanines such as o-, m- or p-halophenylalanine; o-, m- or p-aminophenylalanine, wherein the amino substitutent may be a primary, secondary, or tertiary amine; o-, m- or p-hydroxylphenylalanine; o-, m- or p-O-alkylphenyalanine wherein alkyl represents $C_1$ to $C_4$ alkyl; o-, m- or p-O-acylphenylalanine;

o-, m- or p-S-alkylphenylalanine wherein alkyl represents $C_1$ to $C_4$ alkyl; and the like.

In a preferred embodiment, Xaa is an L-α-amino acid such as serine, diaminobutyric acid, arginine, histidine, tyrosine, or a substituted phenylalanine. More preferably, Xaa is an aromatic an L-α-amino acid such as tyrosine, a substituted tyrosine residue such as iodotyrosine, bromotyrosine, chlorotyrosine, O-alkyl-tyrosine where alkyl represents $C_1$ to $C_8$ alkyl, hydroxyltyrosine, aminotyrosine, and the like, or a substituted phenylalanine residue. Most preferably, Xaa is a substituted phenylalanine residue wherein the substitutions include halogen, amino, hydroxyl, NH-alkyl wherein alkyl represents $C_1$ to $C_4$ alkyl, NH-acyl, O-alkyl wherein alkyl represents $C_1$ to $C_4$ alkyl, O-acyl, S-alkyl wherein alkyl represents $C_1$ to $C_4$ alkyl, SO-alkyl and $SO_2$-alkyl wherein alkyl represents $C_1$ to $C_4$ alkyl, $SO_3$ H, $CO_2H$ $CO_2$-alkyl wherein alkyl represents $C_1$ to $C_4$ alkyl, CONH-alkyl wherein alkyl represents $C_1$ to $C_4$ alkyl. Specific embodiments of such substituted phenylalanine residues include 4-fluorophenylalanine, 4-chlorophenylalanine, 4-bromophenylalanine, 4-iodophenylalanine, 4-nitrophenylalanine, 4-aminophenylalanine, $N^4$-$R^{12}$-4-aminophenylalanine, $N^4$-$R^{12}$, $N^4$ -$R^{13}$-4-aminophenylalanine, or 3- $R^{13}$-4-aminophenylalanine where $R^{12}$ is $C_1$ to $C_4$ alkyl and $R^{13}$ is selected from the group consisting of H, $C_1$ to $C_4$ alkyl amino, hydroxyl, NH-alkyl wherein alkyl represents $C_1$ to $C_4$ alkyl, NH-acyl, O-alkyl wherein alkyl represents $C_1$ to $C_4$ alkyl, O-acyl, S-alkyl wherein alkyl represents $C_1$ to $C_4$ alkyl, SO-alkyl and $SO_2$ -alkyl wherein alkyl represents $C_1$ to $C_4$ alkyl, $SO_3$ H, $CO_2$ H, $CO_2$ -alkyl wherein alkyl represents $C_1$ to $C_4$ alkyl, CONH-alkyl wherein alkyl represents $C_1$ to $C_4$ alkyl. The structures of exemplary most preferred substituted phenylalanine residues for use in this preferred chelators are set forth below in formula (XI-XIV).

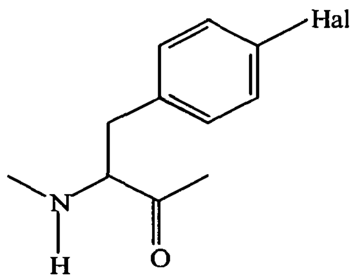

(XI)

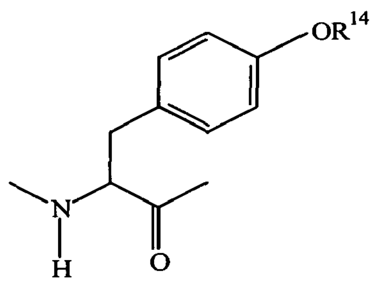

(XII)

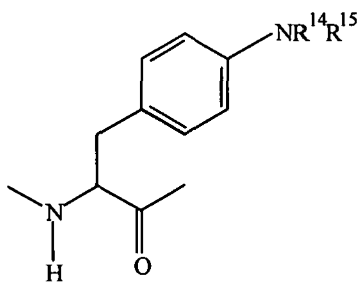

(XIII)

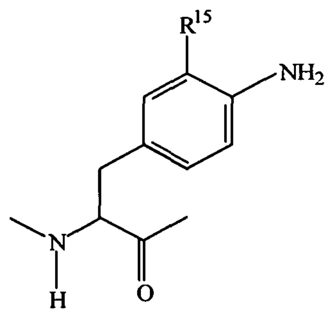

(XIV)

wherein $R^{14}$ and $R^{15}$ are each independently H, a straight chain $C_1$ to $C_4$ alkyl group, a branched chain $C_1$ to $C_4$ alkyl group, or an aryl group and Hal is F, Cl , Br or I. In accordance with the invention, the carboxyl terminal amino acid of the chelators may be in carboxylic acid form or in amidated form, or alternatively, in the form of a .beta.-aminol.

The chelating moieties mentioned above and in particular the preferred peptide based chelating moieties can optionally comprise one or more protected side-chain residues. The side-chains are protected during the synthesis of the compound comprising a cyclized peptide and a metal chelating residue to avoid coupling and/or derivatisation of side-chain functional groups.

In a particular preferred embodiment of the compound of the present invention the metal chelating residue is selected from the group consisting of

a) -βDap-Phe-Cys-Thr-Ser;
b) -βDap-Tyr-Cys-Thr(ol);
c) -βDap-Phe(4-F)-Cys-Thr(ol);
d) -βDap-Phe(4-$NH_2$)-Cys-Thr-Ser;
e) -βDap-Dab-Cys-Thr;
f) -βDap-Phe(4-NH2)-Cys-Thr
g) -βDap-Phe(4-NH2)-Cys-Thr(ol);
h) -βDap-His-Cys-Thr(ol);
i) -βDap-Arg-Cys-Thr(ol);
j) -βDap-Gly-Cys-Lys-$NH_2$;
k) -βDap-Ser-Cys-Thr(ol);
l) -βDap-Dab-Cys-Thr(ol);
m) -βDap-Gly-Cys-Thr(ol);
n) -βDap-Dab-Cys-Ser(ol);
o) -βDap-Ser-Cys-Thr-NH($CH_2CH_2O)_2$ $CH_2CH_2$NH;
p) -βDap-Om-Cys-Thr(ol);
q) -βDap-Dap-Cys-Thr(ol);
r) -βDap-Lys-Cys-Thr(ol); and
s) -βDap-Lys-Cys-NH.

Again, the preferred chelating moieties optionally comprise one or more protected side-chain residues, if required.

In accordance with the invention, the side chain residue of $X^5$ and/or $X^6$, preferably $X^5$ may be linked to a metal chelator. Preferably through a side chain nitrogen, sulfur, or oxygen atom. As set out above linkage may be direct or indirect through intervening atoms or amino acid residues. In a preferred embodiment, the linkage is through -$CH_2$CO-. Such linkages may be accomplished via the alkylation of these atoms with moieties containing reactive electrophiles such as alkyl halides. These atoms may also be reacted with chelators containing isocyanates, isothiocyanates, or activated carboxylic esters. An appropriately protected $X^5$ and/or $X^6$ residue may also be linked to a metal chelator through a side chain carbon by forming a Wittig or Emmons-Horner reagent on the side chain and reacting this with an aldehydo functionality on an appropriately protected chelator. The resulting double bond linkage can be left as is or subsequently reduced to yield saturated hydrocarbon linkage.

Those of skill will recognize that most metal ions may be chelated to the above-mentioned metal ion chelators and ligand/coligand radiometal binding moieties. Any metal ion capable of generating a signal may be chelated to the compounds of the present invention, thus forming a metal ion complex with the compound of the invention. Suitable metal ions include radioactive metal ions, fluorescent metal ions, paramagnetic metal ions, heavy metals, rare earth ions suitable for use in computerized tomography, and the like. Radioactive metal ions or radionuclides are preferred. A wide variety of metals which can be employed in radiotherapeutic or radiodiagnostic approaches are known in the art and comprise without limitation $^{186}$Re, $^{188}$Re, $^{212}$Bi, $^{213}$Bi, $^{90}$Y, $^{153}$Sm, $^{47}$Sc, $^{67}$Ga, $^{94m}$Tc, $^{99m}$Tc, $^{67}$Cu, $^{111}$In, $^{166}$Ho, $^{223}$Ra, and $^{225}$Ac.

On the other hand some one of skill in the art will recognize that the compound of the present invention (with or without a metal chelator attached) can also be labelled with covalently coupled radioisotopes, including halogens and in particular $^{18}$F, $^{125}$I, $^{131}$I, $^{123}$I and $^{211}$At. In a preferred embodiment one or more of these radioisotopes are coupled through a covalent bond to the compounds of the present invention. In principal the radioisotope can be coupled to any moiety within the compound, which is capable of forming a bond to the respective radioisotope. It is, however, preferred that such compounds are coupled to amino acids. The amino acid can be part of the amino acids forming the constrained backbone cyclized peptides or can be one of the to further groups which are coupled to $X^5$ and/or $X^6$. A particular preferred amino acid for the coupling of radioisotopes is tyrosine. Thus, in one preferred embodiment a polypeptide chain, e.g. a metal chelating polypeptide, attached to either $X^5$ and/or $X^6$ is modified by the introduction of $^{18}$F, $^{125}$I, $^{131}$I, $^{123}$I and/or $^{211}$At.

In preferred compounds of the present invention $X^3$, $X^4$, $X^5$ and $X^6$ have the preferred meaning as indicated above, i.e. cyclo[1Na1-DTrp-Lys-Thr-Met-(NMe)Phe]; cyclo[Trp-DTrp-Lys-Thr-Met-(NMe)Phe]; cyclo[1Na1-DTrp-Lys-Val-Met-(NMe)Phe]; cyclo[Phe(4-F)-DTrp-Lys-Thr-Met-(NMe)Phe]; cyclo[Tyr-DTrp-Lys-Val-Met-(NMe)Phe]; cyclo [1Na1-DTrp-Lys-Thr-Lys(GlyMeDOTA)-(NMe)Phe]; cyclo[Tyr-DTrp-Lys-Thr-Met-(NMe)Phe]; cyclo[2Nal-DTrp-Lys-Thr-Met-(NMe)Phe]; cyclo[Tyr-DTrp-Lys-Thr-Met-Tpi]; cyclo[Tyr-Dtrp-Lys-BAla(cyclopropyl)-Met-(NMe)Phe]; cyclo[Tyr-DTrp-Lys-Dpr-Met-(NMe)Phe]; cyclo[ThioPro-DTrp-Lys-Thr-Met-Phe]; cyclo[DiphenylAla-DTrp-Lys-Thr-Met-(NMe) Phe]; cyclo[(4)Pal-DTrp-Lys-Thr-Met-(NMe)Phe] and $X^5$ and/or $X^6$, preferably $X^5$ is linked directly or indirectly, preferably directly, to a metal chelating residue. In that context it is particularly preferred that the metal chelating residue has the preferred meanings as outlined above under a) through k) and even more preferred that the metal chelating moiety has

the meaning -βDap-Xaa-Cys-Zaa-A, wherein Xaa, Zaa and A have the meaning as outlined above. In this context any metal ion may be bound to these compounds, however, preferred metals are γ-emitting radionuclides such as $^{67}Cu$, $^{67}Ga$, $^{111}In$ and $^{99m}Tc$; β-emitting radionuclides such as $^{90}Y$, $^{186}Re$, or $^{166}Ho$; β/γ-emitting radionuclides such as $^{67}Cu$, $^{47}Sc$, $^{153}Sm$ or $^{188}Re$; positrone-emitting radionuclides such as $^{68}Ga$, $^{94m}Tc$, $^{64}Cu$ or α-emitting radionuclides such as $^{213}Bi$, $^{212}Bi$, $^{225}Ac$ , $^{223}Ra$. Most preferably $^{99m}Tc$, $^{188}Re$ and/or $^{186}Re$ are complexed to the compounds of the present invention.

The compounds of the invention may also be complexed with non-radioactive metals such as rhenium, using methods similar to those set forth below. Compounds of the invention complexed to metals can be formed according to known methods. For example, a salt of $^{98m}Tc$ pertechnetate, $^{188}Re$ perrhenate or $^{186}Re$ perenate may be reacted with a compound in the presence of a reducing agent such as dithionite ions, stannous ions or ferrous ions. A particular preferred reducing agent is stannous chloride. Alternatively, complexes and chelats may be formed by ligand exchange, wherein the compound of the invention is reacted with the preformed labile complex of $^{99m}Tc$, $^{188}Re$ or $^{186}Re$ and another compound known as a transfer ligand. In this process any transfer ligand may be used, for example, tartrate, citrate, gluconate, glucoheptonate, manitole, and the like. Exemplary methods for complexes and the compounds of the invention with $^{99m}Tc$ and $^{188}Re$ are set forth in example 4. In general, an appropriate quantity of a compound of the invention is introduced into a vial containing a reducing agent, such as stannous chloride, in an amount sufficient to label the agent with $^{99m}Tc$, $^{188}Re$ or $^{186}Re$. Generally, more reducing agent is required to effect labelling with $^{188}Re$ or $^{186}Re$ than is required to effect labelling with $^{99m}Tc$.

In principal it is possible to couple any drug, which has a residue or side group which is capable of forming a direct or indirect link to the cyclized peptide of the present invention to form a preferred compound of the present invention. Based on the respective chemical nature of the drug someone of skill in the art can determine an appropriate method of coupling the drug directly or indirectly to the cyclized peptide. Preferably the drug, which is coupled to the compound of the present invention is selected from the group consisting of analgesics; antirheumatics; anthelminthics; antiallergics; antianemics; antiarrhythmics; antibiotics; angiogenesis inhibitors; antiinfectives; antidemenics (nootropics); antidiabetics; antidotes; antiemetics; antivertiginosics; antiepileptics; antihemorrhagics; antihypertonics; antihypotonics; anticoagulants; antimycotics; antitussive agents; antiviral agents; beta-receptor and calcium channel antagonists; broncholytic and antiasthmatic agent; chemokines; cytokines, in particular immune modulatory cytokines; mitogens; cytostatics; cytotoxic agents and prodrugs thereof; dermatics; hypnotics and sedatives; immunosuppressants; immunostimulants in particular activators of NF-κB, MAP kinases, STAT proteins and/or protein kinase B/Akt; peptide drugs, protein drugs; in particular hormones and physiological or pharmacological inhibitors of mitogens, chemokines, and cytokines or their respective prodrugs.

It is envisioned to employ the somatostatin analogues of the present invention to target drugs to cells and tissues which show an increased expression of somatostatin receptors, in particular of $SSTR_5$. Such tissues and cells can be found in proliferating tissue and, thus, preferred group of drugs which can be coupled to the compounds of the present invention are cytostatic or cytotoxic drugs. Particular preferred cytostatic or cytotoxic drugs are selected from the group consisting of alkylating substances, anti-metabolites, antibiotics, epothilones, nuclear receptor agonists and antagonists, anti-androgens, anti-estrogens, platinum compounds, hormones and antihormones, interferons and inhibitors of cell cycle-dependent protein kinases (CDKs), inhibitors of cyclooxygenases and/or lipoxygenases, biogenic fatty acids and fatty acid derivatives, including prostanoids and leukotrienes, inhibitors of protein kinases, inhibitors of protein phosphatases, inhibitors of lipid kinases, platinum coordination complexes, ethyleneimenes, methylmelamines, trazines, vinca alkaloids, pyrimidine analogs, purine analogs, alkylsulfonates, folic acid analogs, anthracendiones, substituted urea, and methylhydrazin derivatives. From this different general classes of cytostatic or cytotoxic drugs the following drugs have been successfully employed in the therapy of various tumor diseases and are, thus, particularly preferred: ofacediasulfone, aclarubicine, ambazone, aminoglutethimide, L-asparaginase, azathioprine, bleomycin, busulfan, calcium folinate, carboplatin, carpecitabine, carmustine, celecoxib, chlorambucil, cis-platin, cladribine, cyclophosphamide, cytarabine, dacarbazine, dactinomycin dapsone, daunorubicin, dibrompropamidine, diethylstilbestrole, docetaxel, doxorubicin, enediynes, epirubicin, epothilone B, epothilone D, estramucin phosphate, estrogen, ethinylestradiole, etoposide, flavopiridol, floxuridine, fludarabine, fluorouracil, fluoxymesterone, flutamide fosfestrol, furazolidone, gemcitabine, gonadotropin releasing hormone analog, hexamethylmelamine, hydroxycarbamide, hydroxymethylnitrofurantoin, hydroxyprogesteronecaproat, hydroxyurea, idarubicin, idoxuridine, ifosfamide, interferon α, irinotecan, leuprolide, lomustine, lurtotecan, mafenide sulfate olamide, mechlorethamine, medroxyprogesterone acetate, megastrolacetate, melphalan, mepacrine, mercaptopurine, methotrexate, metronidazole, mitomycin C, mitopodozide, mitotane, mitoxantrone, mithramycin, nalidixic acid, nifuratel, nifuroxazide, nifuralazine, nifurtimox, nimustine, ninorazole, nitrofurantoin, nitrogen mustards, oleomucin, oxolinic acid, pentamidine, pentostatin, phenazopyridine, phthalylsulfathiazole, pipobroman, prednimustine, prednisone, procarbazine, pyrimethamine, raltitrexed, rapamycin, rofecoxib, rosiglitazone, salazosulfapyridine, scriflavinium chloride, semustine streptozocine, sulfacarbamide, sulfacetamide, sulfachlopyridazine, sulfadiazine, sulfadicramide, sulfadimethoxine, sulfaethidole, sulfafurazole, sulfaguanidine, sulfaguanole, sulfamethizole, sulfamethoxazole, co-trimoxazole, sulfamethoxydiazine, sulfamethoxypyridazine, sulfamoxole, sulfanilamide, sulfaperin, sulfaphenazole, sulfathiazole, sulfisomidine, staurosporin, tamoxifen, taxol, teniposide, tertiposide, testolactone, testosteronpropionate, thioguanine, thiotepa, tinidazole, topotecan, triaziquone, treosulfan, trimethoprim, trofosfamide, UCN-01, vinblastine, vincristine, vindesine, vinblastine, vinorelbine, and zorubicin.

In another aspect of the present invention the compound comprising the cyclized peptide further comprises a polypeptide which is either linked directly or indirectly through $X^5$ and/or $X^6$, preferably through $X^5$ to the cyclized peptide. The term "polypeptide" is used to refer to polyamino acids with two or more amino acid residues and, thus, includes peptides, a term which is often used to refer to polyamino acids with two to 100 amino acids and proteins, a term which is often used to refer to polyamino acids with more than 100 amino acids. A polypeptide component can comprise naturally and non-naturally occurring amino acids, in particular alanine, asparagine, cysteine, asparagine, aspartic acid, glutamine, glutamic acid, phenylalanine, glycine, histidine, isoleucine, lysine, leucine, methionine, proline, arginine, serine, threonine, tryptophane, valine, tyrosine, tert-butyl glycine, N-methyl phenylalanine, lysine(GlyMeDOTA), Hcy, Hhc, Pen, Aib, Nal, Aca, Ain, Hly, Achxa, Amf, Aec, Apc, Aes, Aps, Abu, Nva, FD, WD, YD, Cpa, Thp, D-Nal, Dpg, Dab, Nle, ($N-CH_3$)Cys, Orn, ($N-CH_3$)Hcy, ($N-CH_3$)Tyr, ($N-CH_3$)Tty, ($N-CH_3$)Tyr($CH_2CH_2SH$), Thr(OH), Ser(ol), Asp(ol), Glu (ol), Gln(ol), Asn(ol), Phe(4-f), Phe(4-$NH_2$), ε-Lys, γ-Dab, β-Dab.

In a preferred embodiment the polypeptide is selected form the group consisting of a receptor ligand, an antibody, a single chain antibody or a binding fragment of an antibody or single-chain antibody. The term "receptor ligand" refers to poly peptides, which specifically bind to cell surface receptors, i.e. which are natural binding partner of the receptor. The interaction between the receptor and its ligand can have different consequences on one hand it is possible that the ligand and receptor simply act as a tether between, for example, two cells or that the binding can lead to a conformational or functional change of the receptor, which in turn can result in, e.g. activation of an enzymatic function of the receptor or association of the receptor with new and/or different further components within the cell membrane, on the extracellular side or at the cytoplasmatic side of the cell membrane. In this context ligands can have an agonistic or antagonistic effect on the receptor function. Receptor ligands within the meaning of the invention are also modified ligands, which might carry additional N- or C-terminal amino acids or wherein amino acids have been replaced without a significant decrease of binding activity to the receptor. In this context a decrease by more than 90% would be considered significant. Preferably modified ligands show an increase in specific binding. Based on the sequence of a given naturally occurring ligand the skilled person is able to modify the ligand and test the binding strength to the receptor. Preferred receptor ligands of the invention bind to receptors, which are preferentially present on immune cells, e.g. T cell receptor (TcR), CCR5, CXCR4, CD4, CD8, and the like. The skilled person is aware of further receptors that are preferentially expressed on immune cells and ligands to those receptors can all equally be employed in the present invention. If the compounds of the present invention comprise receptor ligands, which are capable of binding to immune cells, the compounds can recruit immune cells, in particular T cells to a diseased area.

The term "antibody" comprises without limitation fully human, humanized, chimeric and xenogenic antibodies. The binding fragment of an antibody is preferably an antibody binding domain fragment, e.g. Fv, Fab, Fab', $F(ab')_2$, Fabc, Facb. The term "single chain" antibody comprises, e.g. single chain Fvs, (scFvs) and diabodies.

In a further embodiment the compound of the present invention can comprise a dye. Such a dye in the context of a somatostatin receptor specific binding component, i.e. the cyclized peptide of the present invention can, for example, allow to label tumor cells *in vivo* and, thus, facilitate the determination of the perimeter of a tumour during a surgical procedure or can be used in imaging techniques employing light of various wave lengths like, e.g. laser imaging. The term "dye" within the meaning of the present invention encompasses substances which are capable of adsorbing light in the visible or invisible light spectrum and which are preferably capable to emit light in the visible or invisible spectrum. Preferred dyes are fluorescent dyes. The skilled person is aware of a large number of dyes which are all suitable for imaging purposes in particular for *in vivo* imaging purposes and which include, for example, fluorescent dyes as described in WO 00/61194, WO 00/71162, WO 01/52746, WO 01/52743 and WO 01/62156. Several methods of coupling dyes to peptide side-chains are known in the art. Preferably, the coupling of dyes is carried out using a reactive side chain of $X^5$ and/or $X^6$. Such groups include, for example, thio, hydroxy, amino or carboxy groups.

A further aspect of the invention is a pharmaceutical composition, comprising a compound of the present invention further pharmaceutically acceptable materials such as, for example, pharmaceutically acceptable salts to adjust the osmotic pressure, buffers, preservatives, carriers and/or excipients. Preferably the pharmaceutical composition is supplied in the form of a pyrogen-free parenterally acceptable pharmaceutical form, which may be an aqueous solution or a lyophilizate for the reconstitution prior to administration. The preparation of such a pharmaceutical composition, having due regard to pH, isotonicity, stability and the like is within the skill in the art. The pharmaceutical composition of the invention may include pharmaceutically acceptable diluents, such as, for example, sodium chloride injection and Ringer's injection. For administration to humans, the composition may be administered in autologous serum or plasma. Supplementary active compounds may also be co-administered with the compounds of the present invention in accordance with the invention.

In addition the pharmaceutical compositions of the invention may optionally contain a stabilizer such as gentisic acid as set forth in U.S. 4,323,000; US 4,233,284; US 4,497,744; US 5,384,113 and/or ascorbic acid as disclosed in US

5,393,512 and US 5,011,676, in WO 97/28181 and in WO 98/33531. Alternatively, hydroquinone stabilizers such as so disclosed in US 4,229,427 may be added to the compounds of the invention. Other compounds such as reductic acid, erythorbic acid, p-amino benzoic acid, 4-hydroxy benzoic acid, nicotinic acid, nicotine amid, 2,5-dihydroxy-1,4-benzene disulfonic acid, tartaric acid, inositol and the like may also be added to the compounds of the present invention in particular if the compounds are complexed to metals.

A further embodiment of the present invention is a kit for preparing radio-metal labelled reagent for use as radiopharmaceutical. The kit of the invention comprises a sealed vial containing a predetermined quantity of a compound of the present invention and a sufficient amount of a reducing agent to label the compound with a metal selected from the group consisting of $^{186}$Re, $^{188}$Re, $^{212}$Bi, $^{213}$Bi, $^{90}$Y, $^{153}$Sm, $^{47}$Sc, $^{67}$Ga, $^{68}$Ga, $^{94m}$Tc, $^{99m}$Tc, $^{67}$Cu, $^{111}$In, $^{166}$Ho $^{223}$Ra, and $^{225}$Ac. Preferably the kit comprises a compound wherein $X^3$, $X^4$, $X^5$ and $X^6$ has the preferred meaning as indicated above in a) through n) and the metal chelating residue is one of the preferred metal chelating residues as indicated above in a) through k) or even more preferred the metal chelating residue according to formula (X)-βDap-Xaa-Cys-Zaa-A, wherein Xaa, Zaa and A have the meaning as outlined above. It is even more preferred that the metal chelator according to formula (X) has the specific structure as indicated in a) to s). It is particular preferred that the reducing agents are chosen to facilitate labelling with $^{186}$Re, $^{188}$Re and $^{99m}$Tc. Stabilizers such as gentisic acid and/or ascorbic acid or any of the stabilizers described above may also be included in kits intended for $^{188}$Re or $^{186}$Re radiolabelling. An appropriate amount of a transfer ligand as described above (such as tartrate, citrate, gluconate, glucoheptonate or manitol, for example) can also be included in the kit. The kit may also contain conventional pharmaceutical adjunct materials such as, for example, pharmaceutical acceptable salts to adjust the osmotic pressure, buffers, preservatives, additional vials and the like. The kit may also contain instructions for radio labelling. The components of the kit may be in liquid, frozen or dry form. In a preferred embodiment, the kit components are provided in lyophilized form.

The kit of the invention may also be embodied in forms suitable for the diagnostic imaging or as a therapeutic agent using a radioisotope of a halogene, including $^{18}$F, $^{211}$At, $^{125}$I, $^{131}$I and preferably $^{123}$I. In this embodiment the kit comprises a sealed vial containing a predetermined quantity of a compound of the invention which is capable of being radiolabelled with a halogene isotope, preferably with $^{123}$I.

A further aspect of the present invention is the use of the compound of the present invention, a pharmaceutical composition of the present or a kit of the present invention for the production of a therapeutic or diagnostic, for the treatment or diagnosis of a somatostatin-responsive disease or a disease characterized by up-regulation of somatostatin receptors. Such diseases which include proliferative diseases, diseases associated with angiogenesis like psoriasis, psoriatic arthritis, rheumatoid arthritis, endometriosis and/or an ocular disease. Such diseases are also described in, for example, Woltering et al. (1997) Investigational New Drugs, 15: 77-86.

The compounds, pharmaceutical compositions and kits of the present invention are particular useful for the diagnosis and/or treatment of proliferative diseases selected from the group consisting of malignomas of the gastrointestinal or colorectal tract, liver, pancreas, kidney, bladder, thyroid, prostate, endometrium, ovary, testes, diuretic, melanoma, dysplastic oral mucosa, invasive oral cancers, small cell and non-small cell lung carcinomas; mammary tumors, e.g. hormone-dependent breast cancers, hormone independent breast cancers; transitional and squamous cell cancers; neurological malignancies including neuroblastomas, gliomas, astrocytomas, osteosarcomas, meningiomas; soft tissue sarcomas; hemangiomas and endocrinological tumors, e.g. pituitary adenomas, pheochromocytomas, paragangliomas, haematological malignancies including lymphomas and leukaemia. Preferred cancers are prostate, small cell lung cancer and diuretic carcinoma.

Preferably radiolabelled complexes of the compound of the invention and compounds comprising cyclized peptides and a cytotoxic or cytostatic drug are used in connection with proliferative diseases, while the former are used for diagnosis and treatment the later are used for treatment only. Radiolabelled embodiments of the compounds of the invention may be used in radioisotope guided surgery as described in WO 93/18797 and in Woltering et al. (1994) Surgery 116: 1139-1147. In a preferred embodiment, a complex of γ-emitting radionuclides such as $^{99m}$Tc and a compound of the invention are used to diagnose in SSTR-expressing tumors and subsequently a complex of a β-emitting radionuclide such as $^{188}$Re or $^{186}$Re and a compound of the present invention is used to treat the tumor. Alternatively, a dye like, for example polymethine dyes, in particular dicarbocyanine, tricarbocyanine, indotricarbocyanine, merocyanine, styryl, squarilium and oxanol dyes and rhodamine dyesphenoxazine or phenothiazin dyes can be used in the diagnosis of diseases followed by therapy with, e.g. a complex β-emitting radionucleotide or with a compound comprising a cyclized peptide and a cytostatic or cytotoxic drug. Particular preferred dyes are cyanine dyes like dicarbocyanine, tricarbocyanine, indotricarbocyanine dyes.

For diagnostic purposes, an effective diagnostic amount of the diagnostic or radiodiagnostic compound of the invention is administered, preferably intravenously. An effective diagnostic amount is defined as the amount of diagnostic or radiodiagnostic compound necessary to effect localization and detection of the label *in vivo* using conventional methodologies such as magnetic resonance, computerized tomography, gamma scintigraphy, SPECT, PET, and the like.

For diagnosis using scintigraphic imaging compounds, preferably $^{99m}$Tc-labeled compounds of the invention are administered in a single unit injectable dose. The compounds, in particular the $^{99m}$Tc-labeled compounds of the

invention may be administered intravenously in any conventional medium for intravenous injection such medium comprise an aqueous saline medium or blood plasma comprising medium. Generally, the unit dose to be administered has a radioactivity of about 0.01 mCi to about 100 mCi, preferably 1 mCi to 50 mCi. The solution to be injected at unit dosage is from about 0.01 ml to about 10 ml.

After intravenous administration of a compound of the present invention, imaging *in vivo* can take place in a matter of a few minutes. However, imaging can take place, if desired, hours or even longer after a dye labelled or radiolabelled compound of the invention is injected into a patient. In most instances, a sufficient amount of the administered dose will accumulate in the area to be imaged within about 0.1 of an hour to permit the taking of images, e.g. scintiphotos. Any conventional method of imaging for diagnostic purposes can be utilized in accordance with this invention.

The compound of the present invention is administered preferably parenteral, and more pref erably intravenous. When the compounds of the invention are used for therapeutic purposes, they preferably comprise a cytotoxic or cytostatic drug and/or a cytotoxic radioisotope, preferably $^{188}$Re. In accordance with the invention, a therapeutically effective amount of a cytotoxic or cytostatic compound means the total amount of the active component of the pharmaceutical composition that is sufficient to show a meaningful patient benefit, i.e., a reduction in the incidence or severity of symptoms attributed to the somatostatin-responsive disease state, as compared to that expected for a comparable group of patients not receiving the compound of the invention. When applied to an individual active ingredient, which is administered alone, the term refers to that ingredient alone. When applied to a combination, the term refers to combined amounts of the active ingredients that result in the therapeutic effect, whether administered in combination, serially, or simultaneously. For the purposes of this invention, the term "therapy" encompasses any therapeutic effect ranging from pain palliation to remission of symptoms associated with the particular somatostatin-responsive disease being treated. In particular in the context of the therapy of proliferative diseases therapy entails pain reduction, tumor ablation, tumor remission and tumor eradication.

When used for radiotherapy, a complex of the compound of the invention and a cytotoxic radioisotope is administered to a mammal, including a human patient, in need of treatment of a somatostatin-responsive disease. In the use of the invention, wherein the compound of the the invention is a radiotherapeutic an amount of cytotoxic radioisotope from about 5 mCi to about 200 mCi may be administered via any suitable clinical route, preferably by intravenous injection or by intratumoral injection. The radiotherapeutic complex of the invention may optionally be administered in combination with a chemotherapeutic drug such as tamoxifen, cisplatin, taxol, anti-angiogenic compounds, and others which were indicated above in the context of preferred drugs for coupling to $X^5$ and/or $X^6$.

If an unlabeled compound is used for therapy, e.g. comprising a cytotoxic or cytostatic drug, the amount of unlabeled compound administered for therapy of a somatostatin-responsive disease will depend upon the nature and severity of the condition being treated, and upon the nature of prior treatments which the patient has undergone. Ultimately, the attending physician will decide the amount of compound with which to treat each individual patient. Initially, the attending physician will administer low doses of the compound and observe the patient's response. Larger doses of the compound may be administered until the optimal therapeutic effect is obtained for the patient, and at that point the dosage is not increased further. It is contemplated that the dosage of unlabeled compound administered in the therapeutic method of the invention should be in the range of about 0.1 $\mu$g to about 100 mg compound per kg body weight. More preferably, the dosage of unlabeled compound administered in the therapeutic method of the invention is in the range of about 0.1 $\mu$g to about 100 $\mu$g compound per kg body weight. The unlabeled compound of the invention may also optionally be administered in combination with a chemotherapeutic drug.

The duration of therapy, whether with a radiopharmaceutical comprising a compound of the invention or with an unlabeled compound of the invention, will vary, depending on the severity of the disease being treated and the condition and idiosyncratic response of each individual patient. It is contemplated that the duration of each administration of the compound of the invention will be in the range of about one to about 120 minutes of continuous intravenous administration. Ultimately the attending physician will decide on the appropriate duration of intravenous therapy using the labelled or unlabeled compounds of the invention, whether administered alone or in combination with other drugs.

## Examples

### Example 1

Synthesis of P2278

The synthesis of cyclized peptides of the present invention were carried out using solid phase chemistry. Such a reaction schema is exemplary indicated in Table 1.

## Table 1

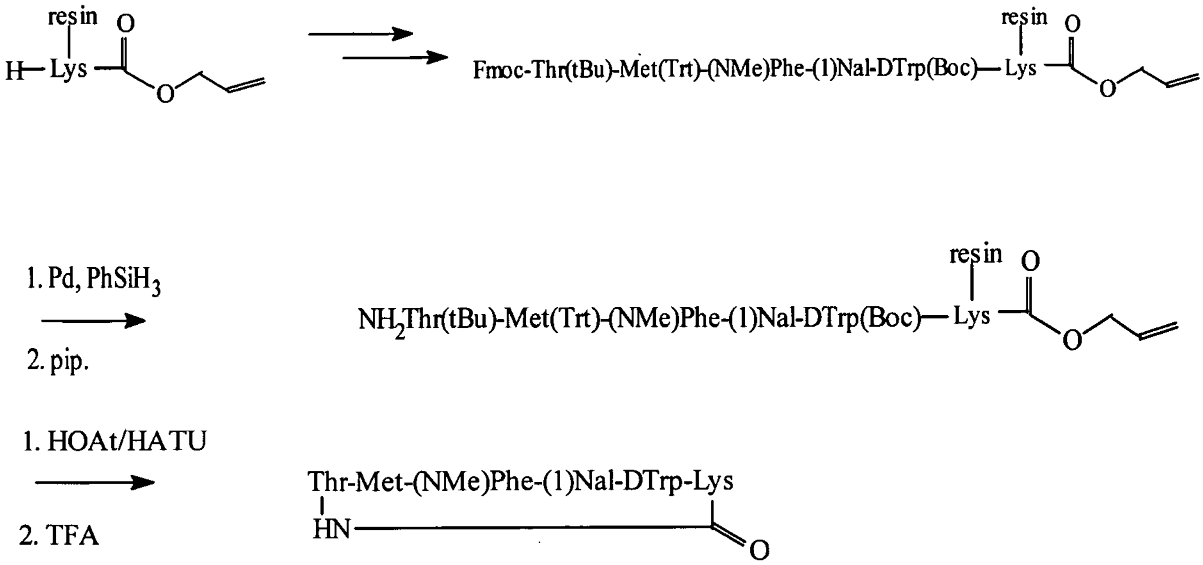


The N-Fmoc protected lysine is tethered to an insoluble, polymer based support via the side-chain amine, and has the carboxylic acid protected as an allyl ester.

### Example 2

Coupling of cyclized Peptides to Metal Chelators or dyes

The coupling of the cyclized peptides to metal chelators and dyes was carried out by standard methods known in the art.

] **Example 3**

Radiolabeling of Compounds of the Invention

Approximately 100 μg of each compound as 100 μl of a 1 mg/ml TFA salt solution dissolved in 0.9% saline was added to a "placebo vial", containing lyophilized 5 mg sodium glucoheptonate dihydrate, 50 μg stannous chloride dihydrate, and 100 μg disodium edetate dihydrate. The vial was then reconstituted with sodium pertechnetate. $^{99m}$Tc (15 to 25 mCi) and saline such that the total volume was 1.1 ml. Following reconstitution, the vials were incubated at 100°C in a water bath for 10-12 minutes.

The purity of the $^{99m}$Tc -labelled compound was determined by reverse-phase analytical HPLC using the following conditions: a Zorbax 300SB C 18, 4μ, 4.6 mm.times.250 mm analytical column was loaded with each radiolabeled compound, and the compound eluted at a solvent flow rate equal to 1.2 ml/min. Gradient elution was performed using a linear gradient of 20-50% Solvent B/Solvent A (Solvent A is 0.1% (v/v) trifluoroacetic acid (TFA) in water and Solvent B is 0.1% (v/v) TFA in 90/10 (v/v) acetonitrile/water) over 20 minutes; followed by a linear gradient of 50-100% Solvent B/Solvent A over four minutes and 100%, Solvent B/Solvent A for three minutes (Method 1).

Radioactive components were detected in the HPLC method using an in-line radiometric detector linked to a computerized data collection and analysis system (Waters Millenium). $^{99m}$Tc -glucoheptonate, $^{99m}$Tc -edetate, and $^{99m}$Tc -pertechnetate elute between one and four minutes under these conditions, whereas the $^{99m}$Tc -labeled compounds eluted after a much greater time. The radiochemical purity (as determined by the % area of the main $^{99m}$Tc product peaks) was ≥80%.

The purity of the $^{99m}$Tc -labeled compound was also determined by TLC quality control analysis. The radiolabeled peptide samples were spotted at the origin of each of two Gelman ITLC-SG strips. One strip each was developed in saturated saline (SAS) and 1:1 (v:v) methanol:1 mol/l ammonium acetate (MAM) and allowed to dry. The SAS strips

were cut at R.sub.f 0.75 and the MAM strip was cut at $R_{0.40}$. The portions of the strips were counted for radioactivity in a dose calibrator, and the percent activity of the top and bottom portions of each strip calculated.

**Example 4**

Binding Experiments comparing binding to $SSTR_2$ and $SSTR_5$

Radiolabeling binding assays were carried out as described in the prior art like, e.g. in Gazal S. et al (2002) J. Med. Chem. 45: 1665-1671. The radioligand binding assays were carried out on membranes prepared from CHO-K1 cells stably expressing individually cloned somatostatin receptors 2 or 5 (SSTR2 or SSTR5). Cells were grown for 2 days for almost confluence and were washed and scraped into 50 mM ice-cold Tris-HCl, pH 7.8, containing 1 mM EGTA, 5 mM $MgCl_2$, 10 mg/mL leupeptin,200 mg/mL bacitracin, 0,1 mM PMSF, and 0,5 mg/mL aprotinin (buffer A) and were centrifuged at 10,000 rpm for 10 min at 4°C (in Sorvall RC 26 Plus ultracentrifuge, rotor SS-34). The pellet was resuspended in buffer A and homogenized with a Polytron PT 1200 homogenizer (Kinematica AG, Switzerland) (setting 2, 3 strokes 10 s each). The homogenate was then centrifuged at 20 000 rpm for 20 min at 4°C. The pellet was resuspended in buffer A and homogenized using the Polytron homogenizer (setting 2, 3 strokes 5 s each). The protein content was determined by Bradford, and the membrane preparation was diluted in buffer A containing 1 mg/mL bovine serum albumin (BSA) to a final 0.2 or 0.4 mg/mL membrane protein (depending on the specific somatostatin receptor used).

The radioligand binding assay was performed in 96 well microtiter plates (Maxisorp plates, Nunc, Denmark). Cell membranes (10 or 20 mg protein) were incubated with the various cyclized peptides indicated in Table 1 & 2, which comprised a lanthanide chelation unit, in a final volume of 250 μL, for 45 min. at room temperature in the presence or absence of competing peptides. Nonspecific binding was defined as the radioactivity remaining bound in the presence of 1 mM somatostatin. At the end of the binding, reaction free radioligand was separated from bound Ligand by rapid filtration through UniFilter GF/C plates preincubated in a solution of 5g/L polyethyleneimine and 1 g/L BSA. The filtration was performed in Filter-Mate Cell Harvester (Packard Instrument Company, U.S.A.). After filtration, the filters were washed several times with cold buffer A containing 1 mg/mL BSA and allowed to dry overnight at room temperature. Then, bound radioactivity was counted. The binding assays were performed in triplicate wells. Data from radioligand binding were used to generate inhibition curves, and $IC_{50}$ values were determined for each of the tested peptides. The saturation binding data were analyzed by the method of Scatchard, and $IC_{50}$ values are expressed as mean ± SEM (standard error of the mean).

The differential binding observed for $SSTR_5$ and $SSTR_2$ are summarized in Table 1.

**Table 1**

| Code No. | Structure | $IC_{50}$ nM $SSTR_2$ | $SSTR_5$ | Ratio 2/5 in % |
|---|---|---|---|---|
| P2278 | cyclo[(1)Nal-DTrp-Lys-Thr-Met-(NMe)Phe] | 0.444 | 1.59 | 27.9 |
| P2269 | cyclo[Trp-DTrp-Lys-Thr-Met-(NMe)Phe] | 0.501 | 3.27 | 15.3 |
| P2329 | cyclo[1Na1-DTrp-Lys-Val-Met-(N-Me)Phe] | 0.437 | 4.17 | 10.5 |
| P2292 | cyclo[Phe(4-F)-DTrp-Lys-Thr-Met-(N-Me)Phe] | 0.152 | 7.61 | 2.0 |
| P2266 | cyclo[Tyr-DTrp-Lys-Val-Met-(NMe)Phe] | 0.256 | 8.12 | 3.2 |
| P2323 | cyclo[1Na1-DTrp-Lys-Thr-Lys(GlyMeDOTA)-(N-Me)Phe] | 37.4 | 9.37 | 399.1 |
| P2265 | cyclo[Tyr-DTrp-Lys-Thr-Met-(NMe)Phe] | 0.139 | 11.6 | 1.2 |
| P2279 | cyclo[2Nal-Dtrp-Lys-Thr-Met-(N-Me)Phe] | 5.58 | 15.0 | 37.2 |
| P2274 | cyclo[Tyr-DTrp-Lys-Thr-Met-Tpi] | 1.22 | 19.9 | 6.1 |
| P2300 | cyclo[Tyr-DTrp-Lys-BAla(cyclopropyl)-Met-(N-Me)Phe] | 0.198 | 20.7 | 0.96 |
| P2267 | cyclo[Tyr-DTrp-Lys-Dpr-Met-(NMe)Phe] | 0.501 | 25.9 | 1.9 |
| P2297 | cyclo[ThioPro-DTrp-Lys-Thr-Met-Phe] | 12.8 | 34.5 | 37.1 |
| P2326 | cyclo[DiphenylAla-DTrp-Lys-Thr-Met-(N-Me)Phe] | 0.177 | 42.2 | 0.42 |
| P2281 | cyclo[(4)Pal-DTrp-Lys-Thr-Met-(NMe)Phe] | 0.154 | 43.3 | 0.36 |

**Comparative Example 1**

The binding assays were carried out as described above, however, the known somatostatin analogues P1839, P2045 and ReP2045 were tested for the differential binding to $SSTR_2$ and $SSTR_5$. The results of the binding are shown in Table 2.

**Table 2**

| Code No. | Structure | $IC_{50}$ nM $SSTR_2$ | $SSTR_5$ | Ratio 2/5 in % |
|---|---|---|---|---|
| P1839 | cyclo[Tyr-DTrp-Lys-Thr-Phe-(NMe)hCys] | 0.032 | 22 | 0.15 |
| P2269 | cyclo[Tyr-DTrp-Lys-Thr-Phe-(NMe)hCys]-$CH_2$CO-βDpr-Phe(4-$NH_2$)-Cys-Thr-Ser-OH | 0.21 | 249 | 0.084 |
| ReP2045 | cyclo[Tyr-DTrp-Lys-Thr-Phe-(NMe)hCys]-$CH_2$CO-βDpr-Phe(4-$NH_2$)-Cys-Thr-Ser-OH-[R=0] | 0.089 | 627 | 0.014 |

Without further elaboration, it is believed that one skilled in the art can, using the preceding description, utilize the present invention to its fullest extent. The above preferred specific embodiments are, therefore, to be construed as merely illustrative, and not limitative of the disclosure in any way whatsoever. In the foregoing examples, all temperatures are set forth uncorrected in degrees Celsius, and all parts and percentages are by weight, unless otherwise indicated.

The entire disclosure[s] of all applications, patents and publications, cited herein are incorporated by reference herein.

The preceding examples can be repeated with similar success by substituting the generically or specifically described reactants and/or operating conditions of this invention for those used in the preceding examples. From the foregoing description, one skilled in the art can easily ascertain the essential characteristics of this invention and, without departing from the spirit and scope thereof, can make various changes and modifications of the invention to adapt it to various usages and conditions.

**Claims**

**1.** Compound comprising a cyclized peptid having a formula (I)

$$\text{cyclo}[X^3\text{-DTrp-Lys-}X^4\text{-}X^5\text{-}X^6] \qquad (I),$$

wherein

$X^3$ is selected from the group consisting of diphenyl-Ala, (1)Nal, (2)Nal, (4)Pal, Phe(4-F), Thioproline, Trp and Tyr;
$X^4$ is selected from the group consisting of βAla(cyclopropyl), diaminopropanoic acid (Dpr), Thr and Val;
$X^5$ is an amino acid containing a side-chain, capable of forming a direct or indirect bond to a metal chelating residue, a polypeptide, a drug or a dye; a natural amino acid; or an unnatural amino acid,
$X^6$ an amino acid containing a side-chain, capable of forming a direct or indirect bond to a metal chelating residue, a therapeutic or a dye; a natural amino acid; or an unnatural amino acid

under the proviso that $X^5$ is cysteine, homo-cysteine or methionine, when $X^3$ has the meaning Tyr and $X^4$ has the meaning Thr.

**2.** Compound according to claim 1, wherein $X^3$ is selected from the group consisting of Tyr and (1)Nal.

**3.** Compound according to claim 1 or 2, wherein $X^4$ is selected from the group consisting of Thr and Val.

**4.** Compound according to one of claims 1 to 3, wherein either $X^5$ or $X^6$ is an amino acid containing a side-chain, capable of forming a direct or indirect bond to a metal chelating residue, a polypeptide, a drug or a dye.

**5.** Compound according to one of claims 1 to 4, wherein at least one of the amino acids $X^3$, $X^4$, $X^5$, or $X^6$ comprise a halogen.

**6.** Compound according to one of claims 1 to 5, wherein $X^5$ is an amino acid containing a side-chain, capable of forming a direct or indirect bond to a metal chelating residue, a polypeptide, a drug or a dye and $X^6$ is a natural amino acid or an unnatural amino acid.

**7.** Compound according to one of claims 1 to 6, wherein the side chain of the amino acid capable of forming a direct or indirect bond to a metal chelating residue, a polypeptide, a drug or a dye is selected from the group consisting of cysteine, homo-cysteine, methionine and Lys(GlyMeDOTA), in particular methionine.

**8.** Compound according to one of claims 1 to 7, wherein the natural or unnatural amino acid is selected from alanine, asparagine, asparagine, aspartic acid, glutamine, glutamic acid, phenylalanine, glycine, histidine, isoleucine, lysine, leucine, proline, arginine, serine, threonine, tryptophane, valine, tyrosine, tert-butyl glycine, N-methyl phenylalanine (NMe)Phe, Hcy, Hhc, Pen, Aib, Nal, Aca, Ain, Hly, Achxa, Amf, Aec, Apc, Aes, Aps, Abu, Nva, FD, WD, YD, Cpa, Thp, D-Nal, Dpg, Nle, (1)Nal, (2)Nal, ($N-CH_3$)Cys, ($N-CH_3$)Hcy, ($N-CH_3$)Tyr, ($N-CH_3$)Tty, ($N-CH_3$)Tyr($CH_2CH_2SH$), Tpi, Thr(OH), Ser(ol), Asp(ol), Glu(ol), Gln(ol), Asn(ol), (4)Pal, Phe(4-F), Phe($4-NH_2$), $\varepsilon$-Lys, $\delta$-Orn, $\gamma$-Dab, and $\beta$-Dap.

**9.** Compound according to one of claims 1 to 8, wherein $X^5$ is selected from the group consisting of cysteine, homo-cysteine, methionine and Lys(GlyMeDOTA), in particular methionine and $X^6$ is selected from the group consisting of (NMe)Phe, Phe and Tpi.

**10.** Compound according to claim 1, wherein $X^3$, $X^4$, $X^5$ and $X^6$ have the meaning as indicated below

a) cyclo[1Nal-DTrp-Lys-Thr-Met-(NMe)Phe];
b) cyclo[Trp-DTrp-Lys-Thr-Met-(NMe)Phe];
c) cyclo[1Na1-DTrp-Lys-Val-Met-(NMe)Phe];
d) cyclo[Phe(4-F)-DTrp-Lys-Thr-Met-(NMe)Phe];
e) cyclo[Tyr-DTrp-Lys-Val-Met-(NMe)Phe];
f) cyclo[1Na1-DTrp-Lys-Thr-Lys(GlyMeDOTA)-(NMe)Phe];
g) cyclo[Tyr-DTrp-Lys-Thr-Met-(NMe)Phe];
h) cyclo[2Na1-DTrp-Lys-Thr-Met-(NMe)Phe];
i) cyclo[Tyr-DTrp-Lys-Thr-Met-Tpi];
j) cyclo[Tyr-Dtrp-Lys-BAla(cyclopropyl)-Met-(NMe)Phe];
k) cyclo[Tyr-DTrp-Lys-Dpr-Met-(NMe)Phe];
l) cyclo[ThioPro-DTrp-Lys-Thr-Met-Phe];
m) cyclo[DiphenylAla-DTrp-Lys-Thr-Met-(NMe)Phe];
n) cyclo[(4)Pal-DTrp-Lys-Thr-Met-(NMe)Phe].

**11.** Compound according to one of claims 1 to 10, wherein the polypeptide is selected from the group consisting of a receptor ligand, an antibody, a single chain antibody or a binding fragment of an antibody or single chain antibody.

**12.** Compound according to one of claims 1 to 11, wherein the metal chelating residue is selected from the group consisting of

a) $C(pgp)^s$-(aa)-$C(pgp)^s$, wherein $(pgp)^s$ is hydrogen or a thiol protecting group and (aa) is any [alpha]- or [beta]-amino acid not comprising a thiol group;
b) a substance according to formula (II) or (III)

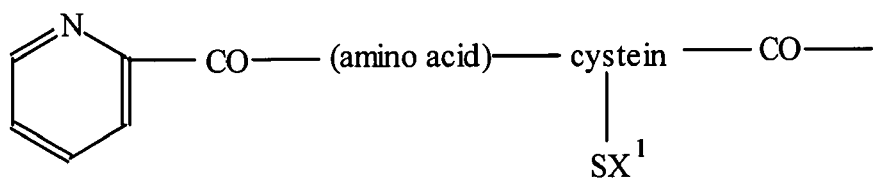

(II)

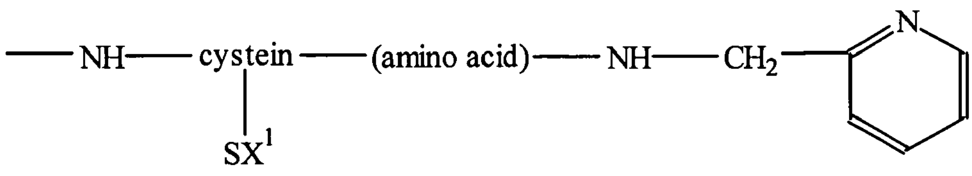

(III),

wherein $X^1$=H or a protecting group;
(amino acid)=any amino acid;
c) a substance according to formula (IV)

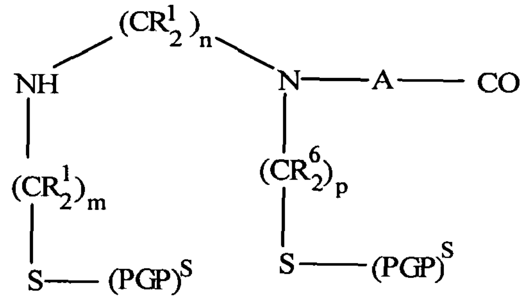

(IV),

wherein each $R^1$ is independently H, $CH_3$ or $C_2H_5$, each $(PGP)^S$ is independently a thiol protecting group or H; m, n and p are independently 2 or 3; A is linear $C_1$-$C_8$ alkyl, substituted linear $C_1$-$C_8$ alkyl, cyclic $C_3$-$C_8$ alkyl, substituted cyclic $C_3$-$C_8$ alkyl, aryl, substituted aryl, or a combination thereof; and
d) a substance according to formula (V)

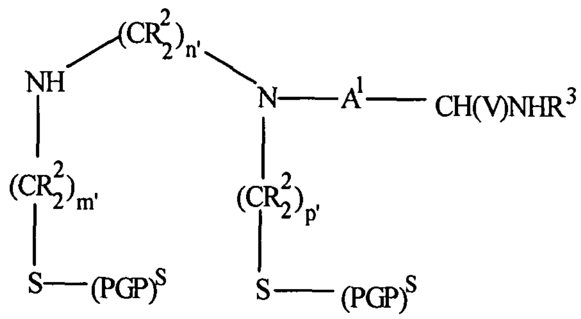

(V),

wherein each $R^2$ is independently H, $CH_3$ or $C_2H_5$; each $(PGP)^S$ is independently a thiol protecting group or H; m', n' and p' are independently 2 or 3; $A^1$ is linear $C_1$-$C_8$ alkyl, substituted linear $C_1$-$C_8$ alkyl, cyclic $C_3$-$C_8$ alkyl, substituted cyclic $C_3$-$C_8$ alkyl, aryl, substituted aryl, or a combination thereof; V is H or a CO link to $X^5$ or $X^6$; $R^3$ is H or covalently linked to $X^5$ or $X^6$;
e) diethylenetriaminepentaacetic acid (DTPA);
f) a derivative of DTPA having a formula (VI)

$$(HOOCCH_2)_2N(CR_2)(CR_2)N(CH_2COOH)(CR_2)(CR_2)N(CH_2COOH)_2 \qquad (VI),$$

wherein each $R^4$ is independently H, $C_1$ to $C_4$ alkyl, or aryl and one $R^4$ is linked to $X^5$ or $X^6$;
g) ethylenediaminetetraacetic acid (EDTA);
h) a derivative of EDTA having a formula (VII)

$$(HOOCCH_2)_2N(CR^5{}_2)(CR^5{}_2)N(CH_2COOH)_2 \qquad (VII),$$

wherein each $R^5$ is independently H, $C_1$ to $C_4$ alkyl, or aryl and one $R^5$ is covalently linked to $X^5$ or $X^6$;
i) 1,4,7,10-tetraazacyclododecanetetraacetic acid and derivatives thereof;
j) a substance according to formula (VIII)

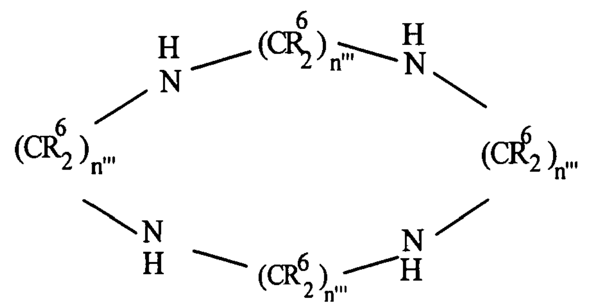

(VIII),

wherein n"' is an integer that is 2 or 3 and where each $R^6$ is independently H, $C_1$ to $C_4$ alkyl, or aryl and one $R^6$ is covalently linked to $X^5$ or $X^6$;
k) a substance according to formula (IX) comprising a single thiol

$$A^2\text{-}CZ^2(B^2)\text{-}\{C(R^7R^8)\}_{n''}\text{-}X^2 \qquad (IX),$$

wherein $A^2$ is H, HOOC-, $H_2NOC$-, -NHOC-, -OOC-, $R_2$ NOC-, $X^2$-NHOC-, $X^2$-OOC-, or $R^9$; $B^2$ is H, SH, -$NHR^{10}$, -$N(R^{10})$-, $X^2$-$NR^{10}$- or $R^9$; $Z^2$ is H or $R^{10}$; $X^2$ is SH, -$NHR^{10}$, -$N(R^{10})$-, $X^2$-$NR^{10}$- or $R^7$; $R^8$, $R^9$ and $R^{10}$ are independently H, straight chain $C_1$-$C_8$ alkyl, branched chain $C_1$-$C_8$ alkyl, or cyclic $C_3$-$C_8$ alkyl; n" is 0, 1 or 2; $R^{11}$ is $C_1$-$C_4$ alkyl, an amino acid, or a peptide comprising 2 to about 10 amino acids; and: (1) where $B^2$ is -$NHR^{10}$, X-$NR^{10}$- or -$N(R^{10})$-, $X^2$ is SH and n" is 1 or 2; (2) where $X^2$ is -$NHR^{10}$, $X^2$-$NR^{10}$-, or -$N(R^{10})$-, $B^2$ is SH and n" is 1 or 2; (3) where $B^2$ is H or R9, $A^2$ is HOOC-, $H_2NOC$-, X-NHOC-, X-OOC-, - NHOC-, or -OOC-, $X^2$ is SH and n" is 0 or 1; (4) where $A^2$ is H or $R^9$, then where $B^2$ is SH, $X^2$ is -$NHR^{10}$, $X^2$-$NR^{10}$-, or -$N(R^{10})$- and where $X^2$ is SH, $B^2$ is -$NHR^{14}$, $X^2$-$NR^{10}$- or -$N(R^{10})$ and n" is 1 or 2; (5) where $X^2$ is H or $R^{10}$, $A^2$ is HOOC-, $H_2NOC$-, -NHOC-, -OOC-, $X^2$-NHOC- or $X^2$-OOC- and $B^2$ is SH; and (6) where $Z^2$ is methyl, $X^2$ is methyl, $A^2$ is HOOC-, $H_2NOC$-, -NHOC-, -OOC-, $X^2$-NHOC- or $X^2$-OOC- and $B^2$ is SH and n" is 0; and
1) a substance according to formula (X)

-βDap-Xaa-Cys-Zaa-A (X),

wherein Xaa is an L-α-amino acid;
Zaa is an α-amino acid, an α-amino acid amide, an aminoethylether, a β-aminol, or a peptide containing from two to ten α-amino acids, said peptide having a carboxyl terminal α-amino acid, α-amino acid amide, aminoethylether, or β-aminol, and A is the amino or carboxyl group of the amino acid, or a protected amino or carboxyl group.

optionally comprising one or more protected side chain residues.

**13.** Compound according to claims 12, wherein the metal chelating residue is selected from the group consisting of:

a) -βDap-Phe-Cys-Thr-Ser;

b) -βDap-Tyr-Cys-Thr(ol);
c) -βDap-Phe(4-F)-Cys-Thr(ol);
d) -βDap-Phe(4-$NH_2$)-Cys-Thr-Ser;
e) -βDap-Dab-Cys-Thr;
f) -βDap-Phe(4-NH2)-Cys-Thr
g) -βDap-Phe(4-NH2)-Cys-Thr(ol);
h) -βDap-His-Cys-Thr(ol);
i) -βDap-Arg-Cys-Thr(ol);
j) -βDap-Gly-Cys-Lys-$NH_2$;
k) -βDap-Ser-Cys-Thr(ol);
l) -βDap-Dab-Cys-Thr(ol);
m) -βDap-Gly-Cys-Thr(ol);
n) -βDap-Dab-Cys-Ser(ol);
o) -βDap-Ser-Cys-Thr-NH($CH_2CH_2O)_2$ $CH_2CH_2$NH;
p) -βDap-Orn-Cys-Thr(ol);
q) -βDap-Dap-Cys-Thr(ol);
r) -βDap-Lys-Cys-Thr(ol); and
s) -βDap-Lys-Cys-NH;

optionally comprising one or more protected side chain residues.

**14.** Compound according to one of claims 1 to 13, further comprising a radiotherapeutic or radiodiagnostic selected from the group consisting of $^{186}$Re, $^{188}$Re, $^{212}$Bi, $^{213}$Bi, $^{90}$Y, $^{153}$Sm, $^{47}$Sc, $^{67}$Ga, $^{68}$Ga, $^{94m}$Tc, $^{99m}$Tc, $^{67}$Cu, $^{111}$In, $^{166}$Ho, $^{223}$Ra, $^{225}$Ac $^{18}$F, $^{125}$I, $^{131}$I, $^{123}$I, and $^{211}$At.

**15.** Compound according to one of claims 1 to 14, wherein $X^3$, $X^4$, $X^5$ and $X^6$ have the meaning as indicated in claim 9 and the metal chelating residue has the meaning as indicated in claim 12.

**16.** Compound according to claim 15, wherein the radiotherapeutic or radiodiagnostic is $^{99m}$Tc, $^{186}$Re or $^{188}$Re.

**17.** Compound according to one of claims 1 to 11, wherein the drug is selected from the group consisting of analgesics; antirheumatics; anthelminthics; antiallergics; antianemics; antiarrhythmics; antibiotics; angiogenesis inhibitors; antiinfectives; antidemenics (nootropics); antidiabetics; antidotes; antiemetics; antivertiginosics; antiepileptics; antihemorrhagics; antihypertonics; antihypotonics; anticoagulants; antimycotics; antitussive agents; antiviral agents; beta-receptor and calcium channel antagonists; broncholytic and antiasthmatic agent; chemokines; cytokines, in particular immune modulatory cytokines; mitogens; cytostatics; cytotoxic agents and prodrugs thereof; dermatics; hypnotics and sedatives; immunosuppressants; immunostimulants in particular activators of NF-κB, MAP kinases, STAT proteins and/or protein kinase B/Akt; peptide drugs, protein drugs; in particular hormones and physiological or pharmacological inhibitors of mitogens, chemokines, and cytokines or their respective prodrugs.

**18.** Compound according to one of claim 17, wherein the cytostatic or cytotoxic drug is selected from the group consisting of alkylating substances, anti-metabolites, antibiotics, epothilones, nuclear receptor agonists and antagonists, anti-androgens, anti-estrogens, platinum compounds, hormones and antihormones, interferons and inhibitors of cell cycle-dependent protein kinases (CDKs), inhibitors of cyclooxygenases and/or lipoxygenases, biogenic fatty acids and fatty acid derivatives, including prostanoids and leukotrienes, inhibitors of protein kinases, inhibitors of protein phosphatases, inhibitors of lipid kinases, platinum coordination complexes, ethyleneimenes, methylmelamines, trazines, vinca alkaloids, pyrimidine analogs, purine analogs, alkylsulfonates, folic acid analogs, anthracendiones, substituted urea, and methylhydrazin derivatives.

**19.** Compound according to one of claim 17, wherein the cytostatic or cytotoxic drug is selected from the group consisting ofacediasulfone, aclarubicine, ambazone, aminoglutethimide, L-asparaginase, azathioprine, bleomycin, busulfan, calcium folinate, carboplatin, carpecitabine, carmustine, celecoxib, chlorambucil, cis-platin, cladribine, cyclophosphamide, cytarabine, dacarbazine, dactinomycin dapsone, daunorubicin, dibrompropamidine, diethylstilbestrole, docetaxel, doxorubicin, enediynes, epirubicin, epothilone B, epothilone D, estramucin phosphate, estrogen, ethinylestradiole, etoposide, flavopiridol, floxuridine, fludarabine, fluorouracil, fluoxymesterone, flutamide fosfestrol, furazolidone, gemcitabine, gonadotropin releasing hormone analog, hexamethylmelamine, hydroxycarbamide, hydroxymethylnitrofurantoin, hydroxyprogesteronecaproat, hydroxyurea, idarubicin, idoxuridine, ifosfamide, interferon α, irinotecan, leuprolide, lomustine, lurtotecan, mafenide sulfate olamide, mechlorethamine, medroxyprogesterone

acetate, megastrolacetate, melphalan, mepacrine, mercaptopurine, methotrexate, metronidazole, mitomycin C, mitopodozide, mitotane, mitoxantrone, mithramycin, nalidixic acid, nifuratel, nifuroxazide, nifuralazine, nifurtimox, nimustine, ninorazole, nitrofurantoin, nitrogen mustards, oleomucin, oxolinic acid, pentamidine, pentostatin, phenazopyridine, phthalylsulfathiazole, pipobroman, prednimustine, prednisone, procarbazine, pyrimethamine, raltitrexed, rapamycin, rofecoxib, rosiglitazone, salazosulfapyridine, scriflavinium chloride, semustine streptozocine, sulfacarbamide, sulfacetamide, sulfachlopyridazine, sulfadiazine, sulfadicramide, sulfadimethoxine, sulfaethidole, sulfafurazole, sulfaguanidine, sulfaguanole, sulfamethizole, sulfamethoxazole, co-trimoxazole, sulfamethoxydiazine, sulfamethoxypyridazine, sulfamoxole, sulfanilamide, sulfaperin, sulfaphenazole, sulfathiazole, sulfisomidine, staurosporin, tamoxifen, taxol, teniposide, tertiposide, testolactone, testosteronpropionate, thioguanine, thiotepa, tinidazole, topotecan, triaziquone, treosulfan, trimethoprim, trofosfamide, UCN-01, vinblastine, vincristine, vindesine, vinblastine, vinorelbine, and zorubicin.

**20.** Compound according to one of claims 1 to 11, wherein the dye is selected from the group consisting of polymethine dyes, in particular dicarbocyanine, tricarbocyanine, indotricarbocyanine, merocyanine, styryl, squarilium and oxanol dyes and rhodamine dyesphenoxazine or phenothiazin dyes.

**21.** Pharmaceutical composition comprising a compound according to any one of claims 1 to 20 and a pharmaceutically acceptable carrier, excipient and/or buffer.

**22.** A kit for preparing a radiopharmaceutical preparation, wherein the kit comprises a sealed vial containing a predetermined quantity of a compound according to claims 1 to 13 and a sufficient amount of a reducing agent to label the compound with a metal selected from the group consisting of $^{186}$Re, $^{188}$Re, $^{212}$Bi, $^{213}$Bi, $^{90}$Y, $^{153}$Sm, $^{47}$Sc, $^{67}$Ga, $^{6a}$Ga, $^{94m}$Tc, $^{99m}$Tc, $^{67}$Cu, $^{111}$In, $^{166}$Ho, $^{223}$Ra, and $^{225}$Ac.

**23.** A kit for preparing a radiopharmaceutical preparation, wherein the kit comprises a sealed vial containing a predetermined quantity of a compound according to claims 1 to 13 wherein the $X^3$, $X^4$, $X^5$ and $X^6$ have the meaning as indicated in claim 10 and the metal chelating residue has the meaning as indicated in claim 13 and a sufficient amount of a reducing agent to label the compound with a metal selected from the group consisting of $^{186}$Re, $^{188}$Re, $^{212}$Bi, $^{213}$Bi, $^{90}$Y, $^{153}$Sm, $^{47}$Sc, $^{67}$Ga, $^{68}$Ga, $^{94m}$Tc, $^{99m}$Tc, $^{67}$Cu,$^{111}$In, $^{166}$Ho, $^{223}$Ra and $^{225}$Ac.

**24.** Use of a compound according to any one of claims 1 to 20 a pharmaceutical composition according to claim 21 or a kit according to claims 22 or 23, for the production of a therapeutic for the treatment of a somatostatin-responsive disease or a disease **characterized by** up-regulation of somatostatin receptors.

**25.** Use of a binding compound producible according to any one of claims 1 to 20 a pharmaceutical composition according to claim 21 or a kit according to claims 22 or 23, for the production of a diagnostic for the diagnosis of a somatostatin-responsive disease or a disease **characterized by** up-regulation of somatostatin receptors.

**26.** Use according to claim 24 or 25, wherein the somatostatin-responsive disease or a disease **characterized by** up-regulation of somatostatin receptors is selected from a proliferative disease, diseases associated with angiogenesis.

**27.** Use according to claim 26, wherein the proliferative disease is selected from the group consisting of malignomas of the gastrointestinal or colorectal tract, liver, pancreas, kidney, bladder, thyroid, prostate, endometrium, ovary, diuretic, testes, melanoma, dysplastic oral mucosa, invasive oral cancers, small cell and non-small cell lung carcinomas; mammary tumors, e.g. hormone-dependent breast cancers, hormone independent breast cancers; transitional and squamous cell cancers; neurological malignancies including neuroblastoma, gliomas, astrocytomas, osteosarcomas, meningiomas; soft tissue sarcomas; hemangioamas and endocrinological tumors, e.g. pituitary adenomas, pheochromocytomas, paragangliomas, haematological malignancies including lymphomas and leukemia.

European Patent Office

# EUROPEAN SEARCH REPORT

Application Number
EP 05 00 9361

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BASS R T ET AL: "IDENTIFICATION AND CHARACTERIZATION OF NOVEL SOMATOSTATIN ANTAGONISTS" MOLECULAR PHARMACOLOGY, BALTIMORE, MD, US, vol. 50, October 1996 (1996-10), pages 709-715, XP002067962 ISSN: 0026-895X MK-678 in Table 1 * the whole document * ----- | 1-3,6,8, 11,21,24 | C07K14/655 C07K7/64 A61K38/12 A61K51/00 |
| D,X | WO 01/44177 A (SCHERING AKTIENGESELLSCHAFT) 21 June 2001 (2001-06-21) cyclo[(N-CH3)Phe-D-Trp-Lys-Thr-Phe-(N-CH3) Hcy](CH2CO.b-Dap.KC.T(ol))* tables 1,2,6 * ----- | 1,3,4, 11-14, 16,21-27 | |
| X | WO 96/04308 A (DIATECH, INC; DEAN, RICHARD, T; MCBRIDE, WILLIAM; LISTER-JAMES, JOHN) 15 February 1996 (1996-02-15) Especially p.9; l.20 * the whole document * ----- | 1-4,6-9, 11-15, 21,24-27 | TECHNICAL FIELDS SEARCHED (IPC) |
| X | WO 95/31221 A (DIATECH, INC) 23 November 1995 (1995-11-23) * the whole document * ----- | 1-4,6-9, 11,12, 14-16, 21-27 | C07K A61K |
| X | WO 95/33497 A (DIATECH, INC) 14 December 1995 (1995-12-14) Especially claim 16, p. 56, l. 31. * the whole document * ----- | 1-4,6-9, 11-16, 21-27 | |
| | -/-- | | |

The present search report has been drawn up for all claims

| Place of search | Date of completion of the search | Examiner |
|---|---|---|
| Munich | 8 February 2006 | Vogt, T |

4

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 05 00 9361

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 95/33498 A (DIATECH, INC) 14 December 1995 (1995-12-14) * the whole document * | 1-4,6-9, 11-16, 21-27 | |
| X | WO 95/00553 A (DIATECH, INC; DEAN, RICHARD, T; MCBRIDE, WILLIAM; LISTER-JAMES, JOHN) 5 January 1995 (1995-01-05) especially claims 32 and 50 * the whole document * | 1-4,6-9, 11,12, 14-16, 21-27 | |
| X | WO 96/11954 A (MERCK FROSST CANADA INC; FLANAGAN, RICHARD, J; DUFOUR, JEAN-MARC; HOGA) 25 April 1996 (1996-04-25) * claims * | 1-4,6-9, 12, 14-16, 21-27 | |
| X | US 4 612 366 A (NUTT ET AL) 16 September 1986 (1986-09-16) * claim 4 * | 1-4,8, 21,24 | TECHNICAL FIELDS SEARCHED (IPC) |
| X | US 5 965 694 A (HIRSCHMANN ET AL) 12 October 1999 (1999-10-12) * claims; sequences 3,5 * | 1-4,6,8, 21,24 | |
| X | WO 97/01579 A (SANDOZ LTD; SANDOZ-PATENT-GMBH; SANDOZ-ERFINDUNGEN VERWALTUNGSGESELLSC) 16 January 1997 (1997-01-16) Especially examples 1, 2, 14, 46 and 48; table on p. 18* examples; table 1 * | 1-4,6,8, 11,12, 14,16, 21-27 | |

-/--

The present search report has been drawn up for all claims

| Place of search | Date of completion of the search | Examiner |
|---|---|---|
| Munich | 8 February 2006 | Vogt, T |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

4

EPO FORM 1503 03.82 (P04C01)

European Patent Office

# EUROPEAN SEARCH REPORT

Application Number
EP 05 00 9361

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | HUANG C-M ET AL: "Targeting delivery of paclitaxel into tumor cells via somatostatin receptor endocytosis" CHEMISTRY AND BIOLOGY, CURRENT BIOLOGY, LONDON, GB, vol. 7, no. 7, July 2000 (2000-07), pages 453-461, XP002243257 ISSN: 1074-5521 * the whole document * ----- | | |
| A | BECKER A ET AL: "RECEPTOR-TARGETED OPTICAL IMAGING OF TUMORS WITH NEAR-INFRARED FLUORESCENT LIGANDS" NATURE BIOTECHNOLOGY, NATURE PUBLISHING, US, vol. 19, April 2001 (2001-04), pages 327-331, XP001168699 ISSN: 1087-0156 * the whole document * ----- | | |
| | | | TECHNICAL FIELDS SEARCHED (IPC) |

The present search report has been drawn up for all claims

| Place of search | Date of completion of the search | Examiner |
|---|---|---|
| Munich | 8 February 2006 | Vogt, T |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................................
& : member of the same patent family, corresponding document

4

EPO FORM 1503 03.82 (P04C01)

## ANNEX TO THE EUROPEAN SEARCH REPORT ON EUROPEAN PATENT APPLICATION NO. EP 05 00 9361

This annex lists the patent family members relating to the patent documents cited in the above-mentioned European search report.
The members are as contained in the European Patent Office EDP file on
The European Patent Office is in no way liable for these particulars which are merely given for the purpose of information.

08-02-2006

| Patent document cited in search report | | Publication date | Patent family member(s) | | | Publication date |
|---|---|---|---|---|---|---|
| WO 0144177 | A | 21-06-2001 | AU | 782873 | B2 | 08-09-2005 |
| | | | AU | 4865901 | A | 25-06-2001 |
| | | | CA | 2383936 | A1 | 21-06-2001 |
| | | | EP | 1208116 | A2 | 29-05-2002 |
| | | | JP | 2003517037 | T | 20-05-2003 |
| | | | US | 6358491 | B1 | 19-03-2002 |
| WO 9604308 | A | 15-02-1996 | AT | 273997 | T | 15-09-2004 |
| | | | AU | 3198495 | A | 04-03-1996 |
| | | | BR | 9508467 | A | 23-12-1997 |
| | | | CA | 2195395 | A1 | 15-02-1996 |
| | | | CN | 1161698 | A | 08-10-1997 |
| | | | DE | 69533399 | D1 | 23-09-2004 |
| | | | DE | 69533399 | T2 | 01-09-2005 |
| | | | EP | 0775160 | A1 | 28-05-1997 |
| | | | ES | 2224131 | T3 | 01-03-2005 |
| | | | JP | 10506880 | T | 07-07-1998 |
| | | | JP | 3117218 | B2 | 11-12-2000 |
| | | | US | 5932189 | A | 03-08-1999 |
| | | | US | 5955426 | A | 21-09-1999 |
| | | | ZA | 9506254 | A | 13-03-1996 |
| WO 9531221 | A | 23-11-1995 | AU | 708797 | B2 | 12-08-1999 |
| | | | AU | 2550095 | A | 05-12-1995 |
| | | | CA | 2190108 | A1 | 23-11-1995 |
| | | | CN | 1155248 | A | 23-07-1997 |
| | | | EP | 0759786 | A1 | 05-03-1997 |
| | | | JP | 10500411 | T | 13-01-1998 |
| | | | ZA | 9503878 | A | 18-01-1996 |
| WO 9533497 | A | 14-12-1995 | AT | 246939 | T | 15-08-2003 |
| | | | AU | 2694495 | A | 04-01-1996 |
| | | | BR | 9507917 | A | 12-08-1997 |
| | | | CA | 2191951 | A1 | 14-12-1995 |
| | | | CN | 1158090 | A | 27-08-1997 |
| | | | DE | 69531502 | D1 | 18-09-2003 |
| | | | DE | 69531502 | T2 | 24-06-2004 |
| | | | DK | 804252 | T3 | 06-10-2003 |
| | | | EP | 0804252 | A2 | 05-11-1997 |
| | | | ES | 2204954 | T3 | 01-05-2004 |
| | | | JP | 3727342 | B2 | 14-12-2005 |
| | | | JP | 10501531 | T | 10-02-1998 |
| | | | KR | 235137 | B1 | 15-12-1999 |
| | | | PT | 804252 | T | 31-12-2003 |
| | | | US | 5976496 | A | 02-11-1999 |
| | | | ZA | 9504548 | A | 15-03-1996 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 05 00 9361

This annex lists the patent family members relating to the patent documents cited in the above-mentioned European search report.
The members are as contained in the European Patent Office EDP file on
The European Patent Office is in no way liable for these particulars which are merely given for the purpose of information.

08-02-2006

| Patent document cited in search report | | Publication date | Patent family member(s) | | | Publication date |
|---|---|---|---|---|---|---|
| WO 9533498 | A | 14-12-1995 | AU | 697048 | B2 | 24-09-1998 |
| | | | AU | 2778395 | A | 04-01-1996 |
| | | | CA | 2191950 | A1 | 14-12-1995 |
| | | | CN | 1154072 | A | 09-07-1997 |
| | | | EP | 0762901 | A1 | 19-03-1997 |
| | | | JP | 10501241 | T | 03-02-1998 |
| | | | KR | 235136 | B1 | 15-12-1999 |
| | | | ZA | 9504547 | A | 24-01-1996 |
| WO 9500553 | A | 05-01-1995 | AU | 701083 | B2 | 21-01-1999 |
| | | | CA | 2167281 | A1 | 05-01-1995 |
| | | | EP | 0720621 | A1 | 10-07-1996 |
| | | | GR | 3035830 | T3 | 31-08-2001 |
| | | | SG | 48977 | A1 | 18-05-1998 |
| | | | SG | 93839 | A1 | 21-01-2003 |
| | | | SG | 90060 | A1 | 23-07-2002 |
| | | | US | 6214316 | B1 | 10-04-2001 |
| WO 9611954 | A | 25-04-1996 | AT | 216708 | T | 15-05-2002 |
| | | | AU | 3602795 | A | 06-05-1996 |
| | | | DE | 69526515 | D1 | 29-05-2002 |
| | | | DE | 69526515 | T2 | 28-11-2002 |
| | | | EP | 0785951 | A1 | 30-07-1997 |
| | | | JP | 3630239 | B2 | 16-03-2005 |
| | | | JP | 10507180 | T | 14-07-1998 |
| | | | US | 5556939 | A | 17-09-1996 |
| US 4612366 | A | 16-09-1986 | CA | 1268899 | A1 | 08-05-1990 |
| | | | DE | 3686099 | D1 | 27-08-1992 |
| | | | DE | 3686099 | T2 | 21-01-1993 |
| | | | EP | 0206118 | A2 | 30-12-1986 |
| | | | JP | 61291599 | A | 22-12-1986 |
| US 5965694 | A | 12-10-1999 | NONE | | | |
| WO 9701579 | A | 16-01-1997 | AT | 210152 | T | 15-12-2001 |
| | | | AU | 714447 | B2 | 06-01-2000 |
| | | | AU | 6515096 | A | 30-01-1997 |
| | | | BR | 9609335 | A | 25-05-1999 |
| | | | CA | 2222524 | A1 | 16-01-1997 |
| | | | CN | 1189166 | A | 29-07-1998 |
| | | | CZ | 9704196 | A3 | 13-05-1998 |
| | | | DE | 69617687 | D1 | 17-01-2002 |
| | | | DE | 69617687 | T2 | 22-08-2002 |
| | | | DK | 835263 | T3 | 02-04-2002 |
| | | | EP | 0835263 | A2 | 15-04-1998 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 05 00 9361

This annex lists the patent family members relating to the patent documents cited in the above-mentioned European search report.
The members are as contained in the European Patent Office EDP file on
The European Patent Office is in no way liable for these particulars which are merely given for the purpose of information.

08-02-2006

| Patent document cited in search report | Publication date | Patent family member(s) | Publication date |
|---|---|---|---|
| WO 9701579 A | | ES 2169251 T3 | 01-07-2002 |
| | | HU 9901455 A2 | 28-09-1999 |
| | | IL 122243 A | 25-09-2005 |
| | | JP 3445796 B2 | 08-09-2003 |
| | | JP 11506108 T | 02-06-1999 |
| | | JP 2003104998 A | 09-04-2003 |
| | | NO 976064 A | 16-02-1998 |
| | | NZ 313147 A | 29-11-1999 |
| | | PL 323943 A1 | 27-04-1998 |
| | | PT 835263 T | 29-04-2002 |
| | | SK 177097 A3 | 05-08-1998 |
| | | TR 9701718 T1 | 21-05-1998 |
| | | TW 491854 B | 21-06-2002 |
| | | US 6225284 B1 | 01-05-2001 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9500553 A
- WO 9533497 A
- US 6183722 B
- WO 01044177 A
- US 5654272 A
- US 5681541 A
- US 5788960 A
- US 5811394 A
- US 5720934 A
- US 5776428 A
- US 5780007 A
- US 5922303 A
- US 6093383 A
- US 6086849 A
- US 5965107 A
- US 5300278 A
- US 5350 A
- US 837 A
- US 5589576 A
- US 5679778 A
- US 5789659 A
- US 6358491 B
- WO 0061194 A
- WO 0071162 A
- WO 0152746 A
- WO 0152743 A
- WO 0162156 A
- US 4323000 A
- US 4233284 A
- US 4497744 A
- US 5384113 A
- US 5393512 A
- US 5011676 A
- WO 9728181 A
- WO 9833531 A
- US 4229427 A
- WO 9318797 A


### Non-patent literature cited in the description


- **VIRGOLINI.** *Eur. J. Clin. Invest.,* 1997, vol. 27, 793-800
- **BLOOM et al.** *Chest,* 1999, vol. 115, 224-232
- **ROHRER, S.P. et al.** *Science,* 1998, vol. 282, 737-740
- **GILON, C.** *J. Med. Chem.,* 1998, vol. 41, 919-929
- **GAZAL S. et al.** *J. Med. Chem,* 2002, vol. 45, 1665-1671
- **G. ZUBAY.** Biochemistry. MacMillen Publishing, 1988, 33
- **NEUGEBAUER et al.** Proceedings of the 11th American Peptide Symposium. 1990, 1020-21
- **WOLTERING et al.** *Investigational New Drugs,* 1997, vol. 15, 77-86
- **WOLTERING et al.** *Surgery,* 1994, vol. 116, 1139-1147
- **GAZAL S. et al.** *J. Med. Chem.,* 2002, vol. 45, 1665-1671